# EUROPEAN PATENT SPECIFICATION

(11) **EP 2 772 537 B1**
(45) Date of publication and mention of the grant of the patent: **30.08.2017**
(21) Application number: 12843544.3
(22) Date of filing: 26.10.2012
(51) Int. Cl.: C12N 9/04, C12P 19/02, C12N 15/09

(54) **POLYOL OXIDASE**
POLYOLOXIDASE
POLYOL OXYDASE

(30) Priority: 27.10.2011 JP 2011236194
(43) Date of publication of application: 03.09.2014
(73) Proprietor: National University Corporation Kagawa University, Takamatsu-shi, Kagawa 760-8521 (JP); Izumoring Co. Ltd., Kagawa 761-0615 (JP); Matsutani Chemical Industry Co., Ltd., Itami-shi, Hyogo 664-8508 (JP)
(72) Inventor: ASADA, Yasuhiko, Kita-gun Kagawa 761-0795 (JP); IZUMORI, Ken, Kita-gun Kagawa 761-0615 (JP)
(74) Representative: TBK
(86) International application number: PCT/JP2012/077783
(87) International publication number: WO 2013/062102

(56) References cited:
- WO-A1-00/65072
- WO-A1-2010/022244
- JP-A- H06 169 764
- JP-A- 2009 511 078
- BOUTELJE J ET AL: "Mannitol dehydrogenase from industrial waste mycelium of Penicillium chrysogenum. Purification, properties and possible use in cofactor regenerating systems in vitro", EUROPEAN JOURNAL OF APPLIED MICROBIOLOGY AND BIOTECHNOLOGY 1983 DE, vol. 17, no. 1, 1983, pages 7-12, XP002741227, ISSN: 0171-1741
- YUKA TOKUNO ET AL.: 'Dojo kara Tanri shita Penicillium sp. ga Seisan suru Shinki To Alcohol Sanka Koso' JAPAN SOCIETY FOR BIOSCIENCE, BIOTECHNOLOGY, AND AGROCHEMISTRY CHUSHIKOKU SHIBU KOENKAI KOEN YOSHISHU 2010, pages 39 A - 18, XP008173303
- WOODYER RD. ET AL.: 'Single-step bioconversion for the preparation of L-gulose and L- galactose' CARBOHYDR. RES. vol. 345, no. 3, 2010, pages 363 - 368, XP026867370

## Description

### Technical Field

The present invention relates to a novel polyol oxidase derived from a microorganism belonging to the genus Penicillium, and a method for efficient production of a rare sugar by utilizing the property of this polyol oxidase such that it is specific for a rare sugar.

### Background Art

It is well known that by utilizing the molecular recognition function of an enzyme to selectively react only with a specific substance, a target substance is produced, or such an enzyme is used in a sensor for detecting a target substance.

An enzyme sensor has advantages that it is simple, prompt, accurate, small, low in cost, and so on as compared with conventionally used general analytical methods such as liquid chromatography and gas chromatography, and therefore is widely used in clinical diagnosis, food analysis, environmental pollution measurement, etc.

As the enzyme sensor, many enzyme sensors have been proposed as follows: for example, an enzyme sensor capable of quantitatively determining xylitol present in a sample simply and specifically in such a manner that xylitol is converted to D-xylose by adding a xylitol oxidase-containing reagent to a sample in which D-sorbitol or glucose is mixed together, and then, a xylose dehydrogenase-containing reagent is allowed to act on the sample, and the resulting D-xylose is detected (PTL 1), and a method for measuring a polyol in a sample for measuring a polyol by allowing a sorbitol oxidase to act on a sample containing at least one type of polyol selected from the group consisting of sorbitol, mannitol, xylitol, and arabitol, and measuring the amount of generated hydrogen peroxide, D-glucose, mannose, xylose, or arabinose, or the amount of consumed oxygen (PTL 2).

As a representative example of the enzyme sensor which has been put into practical use, there is known a blood glucose measuring device which utilizes a glucose oxidase and is used by a diabetic patient. In the blood, a lot of components are mixed together, and it is very difficult to selectively detect glucose among these components. However, by utilizing a glucose oxidase, glucose can be selectively recognized in the blood. This blood glucose measuring device enables a diabetic patient to simply monitor a blood glucose level by drawing the blood by oneself when the diabetic patient gives oneself insulin, and therefore, its market is expanding. The oxidase used also in the blood glucose measuring device consumes oxygen as an electron acceptor when a substrate is oxidized and catalyzes a reaction to generate hydrogen peroxide. The consumption of oxygen can be simply measured using an oxygen electrode, and the generation of hydrogen peroxide can be simply measured using a hydrogen peroxide electrode, and thus, the oxidase is an enzyme suitable for application to the enzyme sensor.

Recently, Japanese society has been aging, and accompanying this, the enhancement of medical technology has been demanded. Further, it is anticipated that food analysis and environment measurement, to which the application of the enzyme sensor has been advanced, are fields in which the need for the enzyme sensor will further increase in future. However, since an oxidase having high substrate specificity is limited at present, it will be necessary to search for a novel oxidase for developing the enzyme sensor in future.

On the other hand, rare sugars are monosaccharides which do not exist at all or exist in a very small amount in nature, and have hardly been studied so far. However, since the mass production of D-psicose and D-allose has become possible, studies of production techniques for rare sugars and studies of physiological actions and chemical properties of rare sugars have been undertaken, and specific physiological actions have been elucidated one after another. When such rare sugars are put into practical use as pharmaceuticals, an enzyme which specifically reacts with rare sugars has been required to be provided. Examples of the physiological activities of rare sugars are shown in the following Table 1.

**[Table 1]**

| Rare sugar | Physiological actions | Usage examples of rare sugar expected to be put into practical use |
|---|---|---|
| D-psicose | Promotion of insulin secretion Inhibition of sugar absorption from intestinal tract (Prevention of diabetes) | Hypoglycemic agent (pharmaceutical preparation or functional food) |
| | Inhibition of secretion of arteriosclerosis promoting factor (arteriosclerosis preventive action) | Hypolipidemic agent (pharmaceutical preparation or functional food) |
| | Fat synthesis inhibitory action | Anti-obesity agent (functional food) |
| D-allose | Reactive oxygen species production inhibitory action | Reactive oxygen species inhibitor (functional food or cosmetic product) |
| | Organ ischemia protective action in brain and liver | Ischemia protectant (pharmaceutical preparation) |
| | Cancer cell growth inhibitory action | Antitumor agent (pharmaceutical preparation) |

With respect also to the utilization of an enzyme for the production of a compound, proposals related to many compounds and enzymes have been made, and for example, recently, by utilizing D-tagatose-3-epimerase (DTE), a technique of mass production of D-psicose or D-allose, each of which is one type of rare sugar, has been established. A method for producing D-allose in which a protein having an L-rhamnose isomerase activity derived from Pseudomonas stutzeri (IPOD FERM BP-08593) is allowed to act on a substrate to isomerize the substrate to D-allose (PTL 3), and a method for producing an aldohexose in which a D-xylose isomerase is allowed to act on a solution containing D-psicose and/or L-psicose to produce D-allose and D-altrose from D-psicose and to produce L-altrose from L-psicose, and one type or two or more types of aldohexoses selected from these D-allose, D-altrose, and L-altrose is/are collected (PTL 4) have been proposed.

### Citation List

### Patent Literature

PTL 1: JP-A-11-346797
PTL 2: JP-A-6-169764
PTL 3: JP-A-2008-109933
PTL 4: JP-A-2002-17392

### Non Patent Literature

NPL 1: Agric. Biol. Chem., 43, 2531-2535 (1979)
NPL 2: J. Biosci. Bioeng., 88, 676-678 (1999)
NPL 3: Shokuhin Eiseigaku Zasshi (Food Hygiene and Safety Science), 49, 82-87 (2008)
NPL 4: Jan. J. Med. Mycol., 45, 55-58 (2004)

### Summary of Invention

### Technical Problem

As described above, it has become known that rare sugars have highly practical physiological activities, and also have the potential of practical application in a wide field of sweeteners, agricultural chemicals, medicines, industrial materials, etc. in addition to this. Therefore, a simple and rapid quantitative determination method is considered to be required in future. However, a micro-quantitative determination method therefor has not been established yet, and thus, it is significant that an oxidase having high specificity for each rare sugar is found and can be utilized in an enzyme sensor. Further, the mass production techniques for all rare sugars have not been established yet, and many of the rare sugars still can be produced in only a small amount through a multistep reaction. Accordingly, if an enzyme capable of producing such a rare sugar by an oxidation reaction is discovered, a novel rare sugar mass production pathway enabling a high yield through a one-step reaction can be created, and thus, it is expected that the study of rare sugars is further advanced.

In such a circumstance, it is well known that a wheat bran culture medium is similar to a condition where a microorganism grows in nature in the respect that the microorganism grows on a surface of a culture medium where the humidity is low. It has been frequently reported that when an actinomycete or a filamentous fungus is cultured using a wheat bran culture medium, a lot of unique enzymes, which are not produced by using a common liquid culture medium or the like, are extracellularly produced. For example, with respect to a lysine oxidase produced by Trichoderma viride (NPL 1) or the like, an oxidase having high substrate specificity is obtained by using a wheat bran culture medium. By studying such conventional techniques, the present invention can provide a novel enzyme which specifically acts on a rare sugar.

An object of the present invention is to provide a polyol oxidase having broad substrate specificity, and another object is to provide a method for efficient production of a rare sugar by utilizing the property of this polyol oxidase. Further, a still another object of the present invention is to find an oxidase having high specificity for each rare sugar and to provide an enzyme sensor for each rare sugar.

### Solution to Problem

A gist of the present invention is a polyol oxidase as defined in the claims, which is described in the following (1) to (3).
(1) A polyol oxidase, which is derived from a microorganism belonging to the genus Penicillium and is specified by the properties listed in the following (a) to (e) :
   (a) it has a stable pH of 6.0 or higher and an optimum reaction pH of from 7.0 to 9.0;
   (b) it has an operating temperature of 50°C or lower and an optimum reaction temperature of 40°C;
   (c) it has a molecular weight of about 113 kDa;
   (d) it specifically recognizes the structure of a polyol in which the OH groups at positions 2 and 3 are in the L-erythro configuration and reacts therewith, and it cannot recognize the structure of a polyol in which the OH group at position 4 is in the L-ribo configuration and does not react therewith; and
   (e) it has substrate specificity for D-arabitol, erythritol, D-mannitol, and D-sorbitol, which are listed in descending order of specificity.
(2) The polyol oxidase according to the above (1), wherein the microorganism belonging to the genus Penicillium is Penicillium sp. KU-1 (Accession Number: NITE BP-1156).
(3) The polyol oxidase according to the above (1) or (2), wherein the polyol oxidase is obtained by culturing the microorganism belonging to the genus Penicillium using wheat bran as a culture medium.

Another gist of the present invention is a method for producing a rare sugar as defined in the claims, which is described in the following (4) to (7).
(4) A method for producing D-mannose, characterized in that the polyol oxidase according to any one of above (1) to (3) is allowed to act on D-mannitol, thereby oxidizing D-mannitol to D-mannose.
(5) A method for producing D-lyxose, characterized in that the polyol oxidase according to any one of the above (1) to (3) is allowed to act on D-arabitol, thereby oxidizing D-arabitol to D-lyxose.
(6) A method for producing L-gulose, characterized in that the polyol oxidase according to any one of the above (1) to (3) is allowed to act on D-sorbitol, thereby oxidizing D-sorbitol to L-gulose.
(7) A method for producing L-erythrose, characterized in that the polyol oxidase according to any one of the above (1) to (3) is allowed to act on erythritol, thereby oxidizing erythritol to L-erythrose.

### Advantageous Effects of Invention

According to the present invention, the following advantageous effects are exhibited.
1. A polyol oxidase having broad substrate specificity can be provided.
2. A polyol oxidase having high reactivity for D-arabitol, erythritol, D-mannitol, and D-sorbitol can be provided.
3. The operation is simple, prompt, and accurate, the size is small, and the cost is low as compared with general analytical methods such as liquid chromatography and gas chromatography, and therefore, the present invention is useful for clinical diagnosis, food analysis, environmental pollution measurement, etc. related to a rare sugar.
4. A rare sugar can be efficiently produced.
5. The present enzyme catalyzes a one-step reaction and also the oxidation reaction is an irreversible reaction, and therefore, it is expected that the present enzyme enables the mass production of a rare sugar in a yield of almost 100%.
6. The following specific production methods for sugars can be provided by utilizing the present polyol oxidase:
   a. a method for producing D-lyxose by using D-arabitol as a starting material;
   b. a method for producing L-erythrose by using erythritol as a starting material;
   c. a method for producing D-mannose by using D-mannitol as a starting material; and
   d. a method for producing L-gulose by using D-sorbitol as a starting material.

### Brief Description of Drawings

[Fig. 1] Fig. 1 shows reactions of oxidases produced by polyol oxidase-producing microorganisms (A strain and B strain).
[Fig. 2] Fig. 2 shows a reaction of an oxidase produced by a D-glucose oxidase-producing microorganism (C strain).
[Fig. 3] Fig. 3 shows the morphology of the C strain on a PDA culture medium.
[Fig. 4] Fig. 4 shows an HPLC analysis of a reaction product obtained by the present enzyme using D-sorbitol as a substrate.
[Fig. 5] Fig. 5 shows the examination results of the substrate-induced production of an enzyme.
[Fig. 6] Fig. 6 shows the effect of the grain coarseness of wheat bran on the productivity of an enzyme.
[Fig. 7] Fig. 7 shows the stability of an enzyme in a crude enzyme solution.
[Fig. 8] Fig. 8 shows TOYOPEARL Butyl-650M column chromatography.
[Fig. 9] Fig. 9 shows TOYOPEARL DEAE-650M column chromatography.
[Fig. 10] Fig. 10 shows Hiprep Q XL column chromatography.
[Fig. 11] Fig. 11 shows HiLoad 16/10 Superdex 200 grade column chromatography.
[Fig. 12] Fig. 12 shows Native-PAGE (left) and SDS-PAGE (right).
[Fig. 13] Fig. 13 shows the results of calculation of a molecular weight using gel filtration column chromatography.
[Fig. 14] Fig. 14 shows the temperature stability of the present enzyme.
[Fig. 15] Fig. 15 shows the pH stability of the present enzyme.
[Fig. 16] Fig. 16 shows the optimum reaction temperature of the present enzyme.
[Fig. 17] Fig. 17 shows the optimum reaction pH of the present enzyme.
[Fig. 18] Fig. 18 shows the structural formulae of polyols (D-form) and the substrate recognition sites therein.
[Fig. 19] Fig. 19 shows the action of the present enzyme on D-arabitol.
[Fig. 20] Fig. 20 shows the reaction of the present enzyme with D-sorbitol.
[Fig. 21] Fig. 21 shows the structure of 18S rDNA and a target amplification region.
[Fig. 22] Fig. 22 shows a photograph of agarose gel electrophoresis after PCR.
[Fig. 23] Fig. 23 shows an alignment search of PCR-amplified fragments.
[Fig. 24] Fig. 24 shows a micrograph of a polyol-producing microorganism.

### Description of Embodiments

The present invention relates to a polyol oxidase, which is derived from a microorganism belonging to the genus Penicillium and is specified by the properties listed in the following (a) to (e):
(a) it has a stable pH of 6.0 or higher and an optimum reaction pH of from 7.0 to 9.0;
(b) it has an operating temperature of 50°C or lower and an optimum reaction temperature of 40°C;
(c) it has a molecular weight of about 113 kDa;
(d) it specifically recognizes the structure of a polyol in which the OH groups at positions 2 and 3 are in the L-erythro configuration and reacts therewith, and it cannot recognize the structure of a polyol in which the OH group at position 4 is in the L-ribo configuration and does not react therewith; and
(e) it has substrate specificity for D-arabitol, erythritol, D-mannitol, and D-sorbitol, which are listed in descending order of specificity.

The polyol oxidase of the present invention has remarkable substrate specificity for D-arabitol, erythritol, D-mannitol, and D-sorbitol, and is useful for the production of a rare sugar or the detection of a rare sugar. In particular, it is useful for the production of D-mannose from D-mannitol, D-lyxose from D-arabitol, L-gulose from D-sorbitol, and L-erythrose from erythritol.

The oxidase of the present invention is derived from a strain KU-1 obtained from soil collected from the Miki-cho Ikenobe community center in Kagawa prefecture, Japan, and as will be described in detail in the following paragraph, this strain was found to be a strain belonging to the genus Penicillium. This Penicillium sp. KU-1 strain was domestically deposited on October 26, 2011 in the Patent Microorganisms Depositary of the National Institute of Technology and Evaluation (2-5-8 Kazusakamatari, Kisarazu City, Chiba Prefecture, Japan) under the Accession Number of NITE P-1156, and is available therefrom. When the international application was filed at this time, a request for transfer of the original deposit (NITE P-1156) was made on October 16, 2012 to the international depositary authority in which the strain was originally deposited as described above, and a proof of receipt (NITE BP-1156) with respect to the original deposit was issued on October 25, 2012 by the international depositary authority.

The Penicillium sp. KU-1 strain belonging to the genus Penicillium which produces the above-described polyol oxidase of the present invention is sometimes also referred to as "A strain" in the following description.

Examples of a known polyol oxidase include a sorbitol oxidase and a xylitol oxidase produced by a microorganism belonging to the genus Streptmyces, however, it has been revealed that in these enzymatic reactions using D-sorbitol as a substrate, D-glucose is produced. On the other hand, it has been strongly suggested that an enzyme produced by a microorganism belonging to the genus Penicillium produces (L-) gulose from D-sorbitol and (D-)lixose from D-arabitol. At present, L-gulose is produced from L-sorbose by using an isomerase. Further, D-lyxose is produced from D-glucose through D-arabitol and D-xylose by a multistep reaction. Moreover, since it is a reaction using an isomerase, the yield of D-lyxose produced is low. In this respect, the present enzyme catalyzes a one-step reaction and also the oxidation reaction is an irreversible reaction, and therefore, the present enzyme enables the mass production of such a rare sugar in a yield of almost 100%. Accordingly, the enzyme of the present invention is expected to be applied to a novel production system of a rare sugar.

Hereinafter, the oxidase of the present invention will be described.

### [1. Isolation of Microorganism]

### 1. Experimental Method

### 1.1 Reagent

As Potato Dextrose Agar (PDA) used in a culture medium, one manufactured by Bection, Dickinson and Company was used.

### 1.2 Culture Medium Composition and Culture Conditions

About 5 mL of water was added to about 1 g of soil collected from various places, whereby a soil suspension was prepared. Then, 50 µL of a solution obtained by diluting the supernatant of the suspension to 100-fold was applied onto a PDA culture medium, followed by incubation at 28°C. Among the colonies formed thereon, a strain which was thought to be a filamentous fungus was inoculated into the same culture medium, followed by incubation at 28°C, whereby the strain was isolated.

### 2. Results and Discussion

Soil was collected from an area on the premises of the Kagawa University and an area in Kagawa Prefecture centering around the Kagawa University, and also collected from an area outside Kagawa Prefecture such as a park in Osaka Prefecture. From the collected soil, 139 strains which were thought to be filamentous fungi were isolated. The types of soil microorganisms vary depending on the collected soil, for example, bacteria were mainly included, a lot of colonies of filamentous fungi could be confirmed, and so on, and therefore, it was revealed that the types of soil microorganisms vary depending on the sampling sites. Further, the growth rate of bacteria is faster than that of filamentous fungi, and therefore, bacteria grew and spread throughout the culture medium and it was difficult to isolate filamentous fungi in some soil. It is considered that in order to prevent this, by performing incubation using a culture medium supplemented with an antibiotic such as ampicillin, it becomes possible to efficiently isolate filamentous fungi.

### [Screening for Novel Oxidase]

The filamentous fungi isolated from the soil were cultured using a wheat bran culture medium, and screening for a filamentous fungus which produces and secretes an oxidase in the culture medium was performed.

### 1. Experimental Method

### 1.1 Reagent

As wheat bran in the wheat bran culture medium used as the culture medium, one purchased from JA Kagawa was used, and as Potato Dextrose Broth (PDB), one manufactured by Bection, Dickinson and Company was used. As a buffer, a potassium phosphate buffer (pH 7) was used, and also as both dipotassium hydrogen phosphate and potassium dihydrogen phosphate, those manufactured by Wako Pure Chemical Industries, Ltd. were used. As for sugars used as substrates, as D-glucose, D-mannitol, and D-sorbitol, those manufactured by Nacalai Tesque, Inc. were used, as D-fructose, D-galactose, and D-mannose, those manufactured by Wako Pure Chemical Industries, Ltd. were used, and as allitol, D-tagatose, D-psicose, and D-allose, those obtained from the Kagawa University Rare Sugar Research Center were used. As for amino acids used as substrates, as creatine and creatinine, those manufactured by Wako Pure Chemical Industries, Ltd. were used, and as L-ornithine, L-citrulline, and γ-aminobutyric acid, those manufactured by Sigma-Aldrich Co. were used. As a peroxidase (ABTS) used for the measurement of an oxidase, one manufactured by Wako Pure Chemical Industries, Ltd. was used in each measurement.

### 1.2 Culture Medium Compositions and Culture Conditions

Isolated microbial cells were inoculated into 5 mL of PDB, followed by shaking incubation at 28°C for 1 week. In a 200-mL Erlenmeyer flask, 6 g of wheat bran and 15 mL of water were added and mixed with each other, and the resulting mixture was sterilized in an autoclave and used as a wheat bran culture medium. To the thus prepared wheat bran culture medium, the liquid-cultured microbial cells were added together with the total amount of the culture solution, and the resulting mixture was incubated at 28°C. After two-week incubation, an extract solution was prepared by a method described below and used for the measurement of the oxidase activity.

### 1.3 Preparation of Extract Solution from Culture Medium

To the wheat bran culture medium in which the microbial cells were grown, 20 mL of a 10 mM potassium phosphate buffer (pH 7.0) was added, and the cells were well immersed therein and left to stand on ice for 1 hour, and then, the mixture was squeezed with a gauze pad. Thereafter, centrifugation was performed at 4°C and 12,000 rpm for 18 minutes to remove fine particles, and the resulting solution was used as a crude extract solution.

### 1.4 Substrate Used for Primary Screening

In primary screening, a sugar mixed solution and an amino acid mixed solution were used in consideration of efficiency. Each substrate was adjusted to give a final concentration of 0.5 M. A combination in each mixed solution is shown in Table 2. The crude extract solution in which the activity was detected by the primary screening was subjected to secondary screening using the respective substrates individually at 0.5 M, and the substrate was specified.

**[Table 2]**

| Aldose mixed solution | Ketose mixed solution | Polyol mixed solution |
|---|---|---|
| D-glucose | | |
| D-mannose | D-tagatose | D-mannitol |
| D-galactose | D-psicose | D-sorbitol |
| D-allose | D-fructose | allitol |

| Amino acid mixed solution 1 | Amino acid mixed solution 2 | Amino acid mixed solution 3 |
|---|---|---|
| creatine | L-citrulline | γ-aminobutyric acid |
| creatinine | L-ornithine | |

### 1.5 Measurement Method for Oxidase Activity

As the measurement method for the oxidase activity, a peroxidase method was used. When a D-glucose oxidase is taken as an example, the principle of the peroxidase method is as follows.
[Chem. 1]

D-glucose oxidase D-glucose + O₂ + 2H₂O → D-gluconic acid + 2H₂O₂

peroxidase 2H₂O₂ + ABTSred → ABTSox 2H₂O + O₂

In this method, the activity is measured by colorimetric determination of hydrogen peroxide generated by an oxidation reaction. When hydrogen peroxide generated by an oxidation reaction with a substrate is reacted with a peroxidase, an oxidation reaction of ABTS is catalyzed to generate oxidized ABTS. The oxidized ABTS develops a blue color, and therefore, by visual observation or measurement of absorbance at 420 nm, the oxidase activity can be detected. In the measurement of the activity, the crude extract solution obtained by extraction from the wheat bran culture medium was used. The composition of the reaction solution for the measurement of the oxidase activity is shown in Table 3. Further, as the control, a reaction solution in which water was added in place of the substrate was also prepared in the same manner. After a reaction was allowed to proceed at room temperature, whether or not the color was developed was confirmed by visual observation, and the crude extract solution added to the reaction solution in which the color was developed was determined to have an oxidase activity.

**[Table 3]**

| | |
|---|---|
| 1 M potassium phosphate buffer (pH 7.0) | 5 µL |
| Peroxidase (10 units/mL) | 10 µL |
| 10 mM ABTS | 10 µL |
| Substrate mixed solution | 10 µL |
| Crude extract solution | 10 µL |
| H₂O | 55 µL |
| Total 100 µL | |

### 2. Results and Discussion

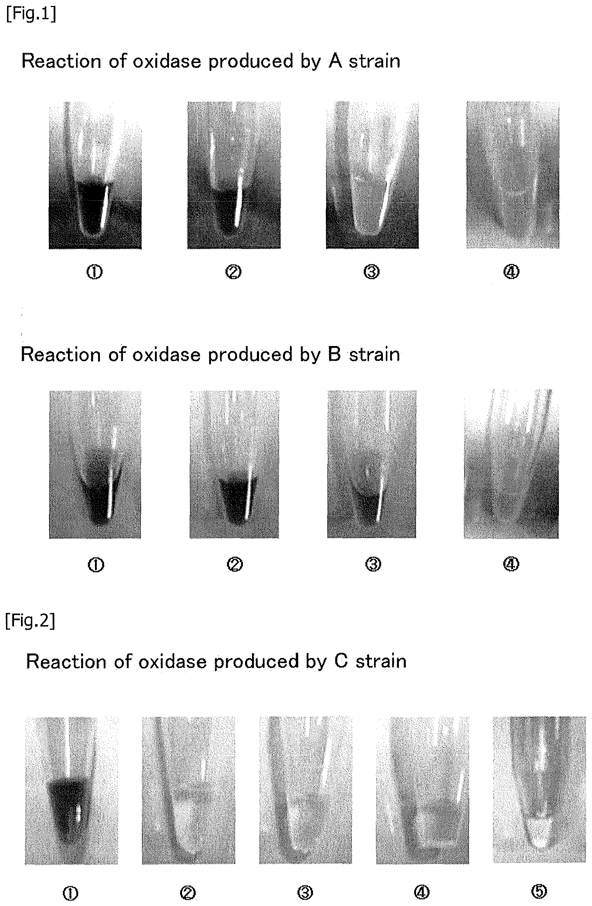

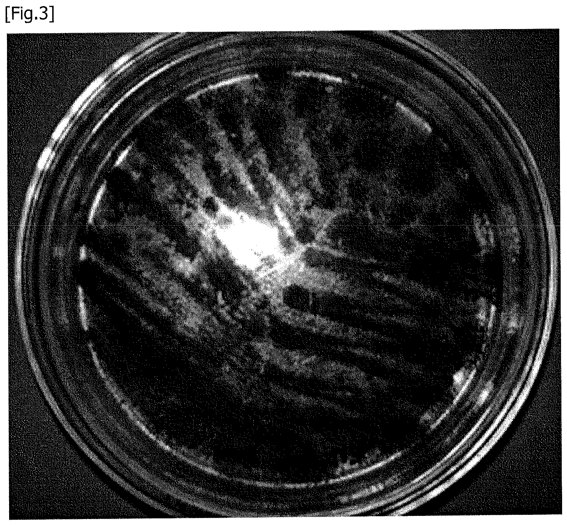

As a result of the screening for an oxidase, three filamentous fungal strains, which are considered to produce an oxidase were found. Among these strains, two strains were microorganisms which produce a polyol oxidase, and one was a strain collected from the Miki-cho Ikenobe community center (hereinafter referred to as "A strain"), and the other was a strain collected from a park in Moriguchi City, Osaka Prefecture (hereinafter referred to as "B strain"). Both of the A strain and the B strain produce a polyol oxidase, and the oxidase of the A strain uses D-sorbitol and D-mannitol as a substrate, but does not show reactivity with allitol, however, the enzyme of the B strain is different in the respect that it uses all of D-sorbitol, D-mannitol, and allitol as a substrate. Further, both enzymes did not react with an aldose or a ketose, and therefore, it was revealed that both enzymes show substrate specificity for a polyol. The enzymatic reactions of these enzymes by the peroxidase method are shown in Fig. 1. Only a few types of polyol oxidases have been reported so far. However, since polyol oxidases produced by two strains and having different substrate specificity could be discovered, there seems to be a possibility that microorganisms which produce a polyol oxidase are unexpectedly widely distributed in nature. The remaining one strain is a strain collected from the soil in Higashi-Osaka City, Osaka Prefecture (hereinafter referred to as "C strain") and produces a D-glucose oxidase. The D-glucose oxidase did not react with other monosaccharides, and therefore was an oxidase showing high substrate specificity for D-glucose. Based on the morphology of the isolated filamentous fungus, the C strain is considered to be Aspergillus niger. It has been already known that A. niger produces a D-glucose oxidase having high substrate specificity, and the enzymatic reaction of this enzyme by the peroxidase method is shown in Fig. 2. Further, the C strain cultured in the PDA culture medium is shown in Fig. 3.

### [2. Examination of Conditions for Production of Polyol Oxidase Produced by A Strain]

The conditions for the production of a polyol oxidase which is produced by the A strain were examined as to whether or not the enzyme was produced in a liquid culture, whether or not the enzyme production was induced by the addition of a substrate, and so on.

### 1. Experimental Method

### 1.1 Microorganism to be Used for Experiment

The A strain which is a polyol oxidase-producing microorganism was used.

### 1.2 Reagent

As a yeast extract used in a culture medium, one manufactured by Nacalai Tesque, Inc. was used.

### 1.3 Culture Medium Composition and Culture Conditions

The microorganism was suspended in 5 mL of sterile water, and a 1 mL portion of the resulting suspension was inoculated into 100 mL of each of liquid culture media obtained by adding (1) PDB and (2) a yeast extract, and mannitol (0.5%) or D-sorbitol (0.5%), followed by shaking incubation at 28°C.

### 1.4 Preparation of Crude Enzyme Solution

Starting from day 2 of the culture, a 500 µL portion was taken out from each culture solution, and centrifuged at 12000 rpm and 4°C for 10 minutes. Then, the supernatant was collected and dialyzed with a 10 mM potassium phosphate buffer (pH 7.0) . The resulting solution was used as a crude enzyme solution.

### 1.5 Measurement Method for Enzyme Activity

In the above-described reaction solution composition, a reaction using D-sorbitol as a substrate was performed, and the presence or absence of the activity was examined by measuring the color development of ABTS through visual observation.

### 2. Results and Discussion

Liquid culture was performed, and the enzyme activity of the crude enzyme solution was measured from day 2 to day 10 of the culture. As a result, the enzyme activity was not detected in either case of the liquid media obtained by adding (1) PDB and (2) a yeast extract, and D-mannitol (0.5%) or D-sorbitol (0.5%). These results suggested that this enzyme is an enzyme favorably produced in a solid culture medium such as a wheat bran culture medium, and also suggested that the substrate-induced production does not occur. It is known that a filamentous fungus generally produces a wide variety of enzymes by solid culture more than by liquid culture, and therefore, this enzyme is considered to be no exception.

### [3. HPLC Analysis of Reaction Product Using D-Sorbitol as Substrate]

An enzymatic reaction was performed using D-sorbitol as a substrate, and the resulting reaction product was analyzed by using HPLC.

### 1. Experimental Method

### 1.1 Microorganism to be Used for Experiment

The A strain which is a polyol oxidase-producing microorganism was used.

### 1.2 Reagent

In a desalting treatment of an HPLC sample, Amberlite manufactured by Organo Co., Ltd. and Diaion manufactured by Mitsubishi Chemical Co., Ltd. were used.

### 1.3 Culture Medium Composition and Culture Conditions

The culture was performed in the same manner as described above.

### 1.4 Preparation of Enzyme Solution

The preparation of an enzyme solution was performed in the same manner as described above.

### 1.5 Enzymatic Reaction

The crude extract solution and D-sorbitol were reacted with each other at room temperature for 24 hours according to the composition shown in Table 4, and the reaction was stopped by boiling. It is considered that dissolved oxygen decreases as the reaction proceeds, and therefore, air was injected into the reaction solution using a pipette. Further, a control was prepared such that immediately after mixing the reaction solution, the reaction was stopped by boiling, and another control was prepared such that the reaction was allowed to proceed for 24 hours in the same manner using a thermally treated crude enzyme solution.

**[Table 4]**

| | |
|---|---|
| 50 mM D-sorbitol | 50 µL |
| Catalase (2.5 units/µL) | 20 µL |
| Crude extract solution | 180 µL |
| Total 250 µL | |

### 1.6 Desalting Treatment and Analysis

After the enzymatic reaction was stopped by boiling, the reaction solution was centrifuged at 13,000 rpm for 10 minutes to remove fine particles, and the supernatant was collected. To the supernatant, a small amount of a mixture obtained by mixing Diaion and Amberlite at a ratio of 1:2 was added, and the resulting mixture was left to stand for 1 hour to desalt the reaction solution. The supernatant thereof was collected and centrifuged at 13,000 rpm for 5 minutes. Thereafter, the resulting solution was placed in a 0.22-µm filter (Ultrafree-MC) and centrifuged at 6000 rpm for 5 minutes. The total amount of the solution passed through the filter was collected and injected into a dedicated HPLC tube (sample cup IA) such that air bubbles do not enter the tube. Then, an analysis was performed by using an autosampler.

### 1.7 Conditions for HPLC

By using GL-C611 column chromatography (Hitachi Chemical Co., Ltd.), detection was performed with a differential refractometer using an aqueous 10⁻⁴ M sodium hydroxide solution as a mobile phase.

### 2. Results and Discussion

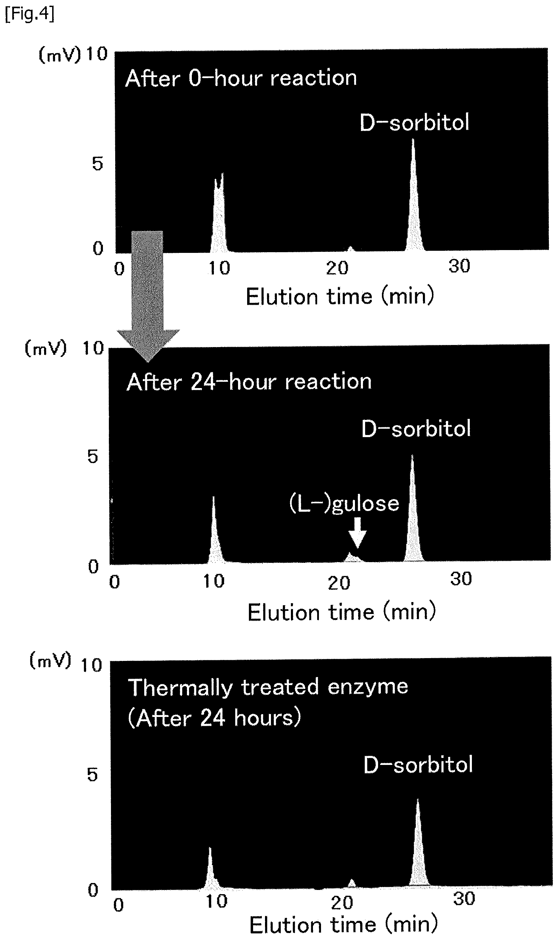

The enzymatic reaction was performed for 24 hours by using D-sorbitol as a substrate, and its reaction product was analyzed by using HPLC. As a result, as shown in Fig. 4, a peak, which did not appear in the case of the reaction solution of the control, was confirmed at around 21 minutes. This corresponds to the elution time of gulose which is a rare sugar. Further, a peak at around 9 minutes is considered to be a salt or the like, and a peak at around 20 minutes corresponds to the elution time of idose. However, similar peaks were confirmed also in the case of the control, and therefore, it is considered that these are not substances derived from the enzymatic reaction. Incidentally, a peak at around 28 minutes is derived from D-sorbitol used as the substrate.

These results strongly suggested that the reaction product of D-sorbitol and the enzyme produced by the A strain is gulose which is a rare sugar. Further, although an optical activity was not measured, when considering that sorbitol was used as the substrate, the product is structurally considered to be L-gulose. Although the oxidation reaction is an irreversible reaction, the amount of (L-) gulose produced after the 24-hour reaction was as small as about 3%. It is considered that this is because the crude enzyme solution was used and also the oxygen concentration in the reaction solution was insufficient.

### [4. Examination of Conditions for Production of Polyol Oxidase Derived from Strain Belonging to Genus Penicillium (A Strain)]

A method capable of more efficiently obtaining a crude enzyme solution of a polyol oxidase derived from the genus Penicillium was examined as to whether or not the enzyme was produced in a liquid culture, whether or not the enzyme production was induced by the addition of a substrate, and so on.

### 1. Experimental Method

### 1.1 Microorganism to be Used for Experiment

A strain which belongs to the genus Penicillium and produces a polyol oxidase (A strain) was used.

### 1.2 Culture Medium Composition and Culture Conditions 1) Liquid Culture Medium

The microorganism was suspended in 5 mL of sterile water, and a 1 mL portion of the resulting suspension was inoculated into 100 mL of each of liquid culture media obtained by adding (1) PDB and (2) a yeast extract, and D-mannitol (0.5%) or D-sorbitol (0.5%), followed by shaking incubation at 28°C.

### 2) Examination of Substrate-Induced Production of Enzyme

The microorganism cultured by shaking in a PDB culture medium at 28°C for 3 days was inoculated in an amount of 5 mL into each of a culture medium containing no D-mannitol and a culture medium containing D-mannitol, followed by static incubation at 28°C.

**[Table 5]**

| | |
|---|---|
| Wheat bran | 20 g |
| H₂O | 40 mL |
| Per 500-mL Erlenmeyer flask | |

**[Table 6]**

| | |
|---|---|
| Wheat bran | 20 g |
| Aqueous 2% D-mannitol solution | 40 mL |
| Per 500-mL Erlenmeyer flask | |

### 3) Effect of Grain Coarseness of Wheat Bran on Productivity of Enzyme

A wheat bran culture medium having each of the following compositions was used, and the microorganism was inoculated and cultured in the same manner as in the case of the addition of a sugar to a wheat bran culture medium. The ratio of wheat bran to water is shown in Tables 7 and 8.

**[Table 7]**

| | |
|---|---|
| Wheat bran | 20 g |
| H₂O | 40 mL |
| Per 500-mL Erlenmeyer flask | |

**[Table 8]**

| | |
|---|---|
| Wheat bran | 20 g |
| H₂O | 30 mL |
| Per 500-mL Erlenmeyer flask | |

### 1.3 Preparation of Enzyme Solution

### 1) Liquid Culture Medium

Starting from day 2 of the culture, a 500 µL portion was taken out from each culture solution, and centrifuged at 12, 000 rpm and 4°C for 10 minutes. Then, the supernatant was collected and dialyzed with a 10 mM potassium phosphate buffer (pH 7.0) . The resulting solution was used as a crude enzyme solution.

### 2) Examination of Substrate-Induced Production of Enzyme

In the same manner as described above, a crude enzyme solution was obtained. However, the buffer was changed to a 10 mM potassium phosphate buffer (pH 8.0).

### 3) Effect of Grain Coarseness of Wheat Bran on Productivity of Enzyme

Extraction was performed in the same manner as the examination of the substrate-induced production of the enzyme, and a crude enzyme solution was obtained.

### 1.4 Measurement Method for Enzyme Activity 1) Liquid Culture Medium

In the above-described reaction solution composition, a reaction using D-mannitol as a substrate was performed, and the presence or absence of the activity was examined by measuring the color development of ABTS through visual observation.

### 2) Examination of Substrate-Induced Production of Enzyme

The measurement of the activity of the present enzyme was performed by measuring an increase in absorbance at 420 nm using the same peroxidase method as described above. The enzymatic reaction was initiated by adding D-mannitol, and the absorbance was measured over time for minutes under the reaction conditions of 30°C using a spectrophotometer U-2010 manufactured by Hitachi Co., Ltd. The reaction was performed by preparing the reaction solution so that the total amount thereof was 1 mL as shown in Table 9. "1 unit" was defined as the amount of the enzyme required to increase the absorbance at 420 nm by 1 for 1 minute.

**[Table 9]**

| | |
|---|---|
| 1 M potassium phosphate buffer (pH 8.0) | 50 µL |
| Peroxidase (10 units/mL) | 100 µL |
| 10 mM ABTS | 100 µL |
| 0.5 M D-mannitol | 100 µL |
| Crude extract solution | 100 µL |
| H₂O | 550 µL |
| Total 1 mL | |

### 3) Effect of Grain Coarseness of Wheat Bran on Productivity of Enzyme

The measurement was performed in the same manner as in the case of the addition of a sugar to a wheat bran culture medium.

### 2. Results and Discussion

### 1) Liquid Culture Medium

The enzyme activity of the crude enzyme solution was measured from day 2 to day 10 of the culture. As a result, the enzyme activity was not detected in either case of the liquid media. These results suggested that this enzyme is an enzyme favorably produced in a solid culture medium such as a wheat bran culture medium. Further, the growth rate in the liquid culture medium was equal to or faster than that in the solid culture medium, and therefore, it was suggested that the conditions for the production of the enzyme does not depend on the mycelial growth.

### 2) Examination of Substrate-Induced Production of Enzyme

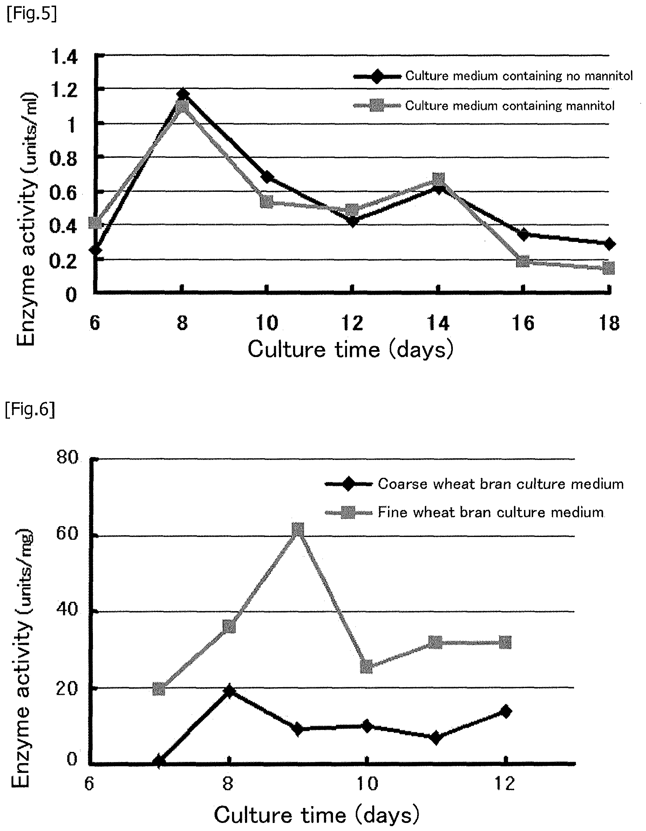

The extraction of the crude enzyme solution was performed from day 6 to day 18 of the culture and the measurement of the enzyme activity was performed, and the results are shown in Fig. 5. Since a significant difference in enzyme activity was not observed, it was suggested that the production of this enzyme is not induced by the addition of a substrate. Further, since wheat bran contains a sugar serving as an inducer such as mannitol, there is no denying the possibility that even if a substrate is further added to the wheat bran culture medium, further induction is not observed.

### 3) Effect of Grain Coarseness of Wheat Bran on Productivity of Enzyme

The extraction of the crude enzyme solution was performed from day 6 to day 12 of the culture and the measurement of the enzyme activity was performed, and the results are shown in Fig. 6. These results showed that there was a 2.6 times difference between the peak enzyme activities, and it was revealed that the enzyme can be more favorably produced in the case of culturing this microorganism using fine wheat bran. Further, the peak of the enzyme activity appeared around day 9 of the culture. According to these results, by using the crude enzyme solution extracted from the culture solution obtained by culturing the microorganism for 9 days in the fine wheat bran culture medium, the purification of the present enzyme was attempted.

### [5. Examination of Stability of Enzyme Using Crude Enzyme Solution]

Prior to purification, the stability of the present enzyme in the crude enzyme solution was examined.

### 1. Experimental Method

### 1.1 Microorganism to be Used for Experiment

A strain which belongs to the genus Penicillium and produces a polyol oxidase (A strain) was used.

### 1.2 Reagent

As ammonium sulfate used in ammonium sulfate fractionation, one manufactured by Nacalai Tesque, Inc. was used.

### 1.3 Culture Medium Composition and Culture Conditions

In a 500-mL Erlenmeyer flask, 20 g of wheat bran and 40 mL of water were added and mixed well using a spatula. Then, the flask was sealed with a cotton plug, and the mixture in the flask was sterilized in an autoclave and used as a wheat bran culture medium. A culture solution obtained by shaking culture using PDB for 3 days was inoculated in an amount of 5 mL into the flask, followed by static incubation at 28°C for 10 days.

### 1.4 Preparation of Enzyme Solution

A crude extract solution was obtained in the same manner as described above. However, the buffer was changed to a 10 mM potassium phosphate buffer (pH 8.0). A half the crude extract solution was used as the crude enzyme solution, and the residual enzyme activity was measured at times of 0 hour, 5 hours, and 24 hours after the crude enzyme solution was left to stand on ice. The rest of the crude extract solution was used for preparing a 50 to 70% saturated ammonium sulfate fraction by ammonium sulfate precipitation, and the residual enzyme activity was measured at times of 0 hour, 5 hours, and 24 hours after the saturated ammonium sulfate fraction was left to stand on ice in the same manner.

### 1.5 Measurement Method for Enzyme Activity

The enzyme activity was measured in the same manner as described above.

### 2. Results and Discussion

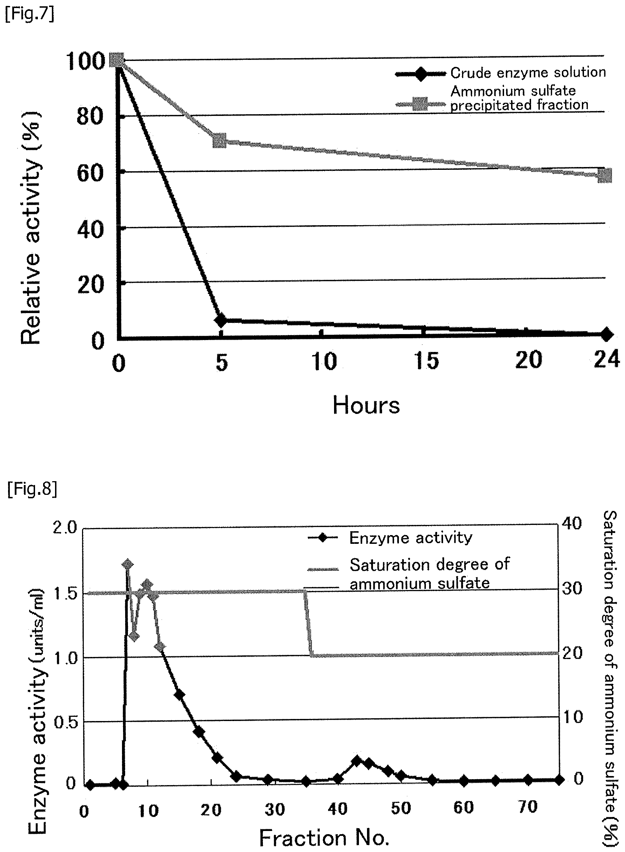

The results of the measurement of the enzyme activity are shown in Fig. 7 and Table 10.

The residual enzyme activity after the crude enzyme solution was left to stand on ice for 5 hours was about 5%, and the enzyme activity was completely lost after 24 hours. However, the 50 to 70% saturated ammonium sulfate fraction prepared by ammonium sulfate precipitation had a residual activity of 60% even after it was left to stand on ice for 24 hours. This suggested that in the crude enzyme solution, a high protease activity exists, and the protease can be removed to some extent by ammonium sulfate fractionation.

**[Table 10]**

| | Relative activity (%) | | |
|---|---|---|---|
| | After 0 hour | After 5 hours | After 24 hours |
| Crude enzyme | 100 | 6 | 0 |
| Ammonium sulfate precipitated fraction (50 to 70%) | 100 | 70 | 57 |

### [6. Purification of Polyol Oxidase Derived from Microorganism Belonging to Genus Penicillium]

### 1. Experimental Method

### 1.1 Microorganism to be Used for Experiment

A strain which belongs to the genus Penicillium and produces a polyol oxidase (A strain) was used.

### 1.2 Culture Medium Composition and Culture Conditions

In a 500-mL Erlenmeyer flask, 20 g of wheat bran and 30 mL of water were added and mixed with each other. Then, the flask was sealed with a cotton plug, and the mixture in the flask was sterilized in an autoclave and used as a wheat bran culture medium, and the microorganism was cultured at 28°C for 9 days.

### 1.3 Measurement Method for Enzyme Activity

The measurement of the activity of the present enzyme was performed by measuring an increase in absorbance at 420 nm using the same peroxidase method as described above. The enzymatic reaction was initiated by adding D-mannitol, and the absorbance was measured over time for 10 minutes under the reaction conditions of 30°C using a spectrophotometer U-2010 manufactured by Hitachi Co., Ltd. The reaction was performed by preparing the reaction solution so that the total amount thereof was 1 mL as shown in Table 11. "1 unit" was defined as the amount of the enzyme required to increase the absorbance at 420 nm by 1 for 1 minute.

**[Table 11]**

| | |
|---|---|
| 1 M potassium phosphate buffer (pH 8.0) | 50 µL |
| Peroxidase (10 units/mL) | 100 µL |
| 10 mM ABTS | 100 µL |
| 0.5 M D-mannitol | 100 µL |
| Enzyme solution | 100 µL |
| H₂O | 550 µL |
| Total 1 mL | |

### 1.4 Quantitative Determination of Protein

The quantitative determination of a protein was performed by using a protein assay CBB solution manufactured by Nacalai Tesque, Inc. according to the Bradford method. In the preparation of a calibration curve, bovine serum albumin was used as a standard protein. In the measurement of an absorbance, a spectrophotometer U-3200 manufactured by Hitachi Co., Ltd. was used.

### 1.5 Sodium Dodecyl Sulfate-Polyacrylamide Gel Electrophoresis (SDS-PAGE)

SDS-PAGE was performed by adjusting a separation gel at 15% and a concentration gel at 4% according to the Laemmli method. As the molecular weight marker, Protein Marker Low Range manufactured by SIGMA Co., Ltd. was used.

### 1.6 Native-PAGE and Activity staining

Native-PAGE was performed by adjusting a separation gel at 15% and a concentration gel at 4% according to the Davis method. Activity staining was performed in such a manner that the gel after the electrophoresis was washed with water, and then immersed in a solution having the same composition shown in Table 11 in a light shielding state.

### 1.7 Purification of Enzyme

The purification of the enzyme was performed on ice or at 4°C throughout the following procedures (1) to (4). Further, as the buffer, a 10 mM potassium phosphate buffer (pH 8.0) was used.

### (1) Preparation of Crude Enzyme Solution

To the culture medium after culture, 60 mL of the buffer was added and mixed well, and the resulting mixture was left to stand on ice for 1 hour or more. Then, the mixture was squeezed with a gauze pad to extract the enzyme in the culture medium. The resulting extract solution was centrifuged at 4°C and 800 rpm for 10 minutes to remove fine particles, and the resulting solution was used as a crude enzyme solution.

### (2) 50 to 70% Saturated Ammonium Sulfate Fraction

First, ammonium sulfate was added to the crude enzyme solution to bring the solution to 50% saturation and dissolved therein, followed by stirring at 4°C for 3 hours. The resulting solution was centrifuged at 4°C and 8,000 rpm for 10 minutes. The thus obtained supernatant was collected, and ammonium sulfate was added thereto to bring the supernatant to 70% saturation and dissolved therein, followed by stirring overnight at 4°C. The resulting solution was centrifuged at 4°C and 8, 000 rpm for 10 minutes, and the resulting precipitate was dissolved in a buffer brought to 40% saturation with ammonium sulfate.

### (3) TOYOPEARL Butyl-650M Column Chromatography

A sample was subjected to a TOYOPEARL Butyl-650M column (diameter: 2.0 cm, length: 8.0 cm) equilibrated with a buffer brought to 40% saturation with ammonium sulfate. The column was washed with 100 mL of a potassium phosphate buffer brought to 40% saturation with ammonium sulfate, and the enzyme was eluted with buffers in an amount of 100 mL each by stepwise changing the saturation concentration of ammonium sulfate in the buffers to 30% and 20%, and a fraction having an activity was collected. Ammonium sulfate contained in the collected fraction was removed by dialysis overnight with a buffer at 4°C. In the measurement of an increase in absorbance at 420 nm at this time, a spectrophotometer U-3200 manufactured by Hitachi Co. , Ltd. was used, and the absorbance was not measured over time, but the value of an absorbance at one point when one minute passed after the initiation of the reaction was measured.

### (4) TOYOPEARL DEAE-650M Column Chromatography

A sample was subjected to a TOYOPEARL DEAE-650M column (diameter: 1.0 cm, length: 5.0 cm) equilibrated with a buffer. The column was washed with 50 mL of a buffer, and the enzyme was eluted with buffers in an amount of 50 mL each by stepwise changing the concentration of sodium chloride in the buffers to 0 mM, 100 mM, 150 mM, and 200 mM, and a fraction having an activity was collected. Sodium chloride contained in the collected fraction was removed by dialysis overnight with a buffer at 4°C. Also in the measurement of an increase in absorbance at 420 nm at this time, a spectrophotometer U-3200 manufactured by Hitachi Co., Ltd. was used, and the absorbance was not measured over time, but the value of an absorbance at one point when one minute passed after the initiation of the reaction was measured.

### (5) Hiprep Q XL Column Chromatography

A sample was concentrated to about 1 mL by using Amicon Ultra-15 (manufactured by Millipore Co., Ltd.), and the resulting concentrate was subjected to Hiprep Q XL (GE Healthcare) equilibrated with 200 mL of a buffer. The enzyme was eluted by applying a linear concentration gradient of sodium chloride in the buffer from 0 to 0.5 M, and a fraction having an activity was collected. Sodium chloride contained in the collected fraction was removed by dialysis overnight with a buffer at 4°C. Also in the measurement of an absorbance at this time, a spectrophotometer U-3200 manufactured by Hitachi Co. , Ltd. was used, and the value of an absorbance when one minute passed after the initiation of the reaction was measured.

### (6) HiLoad 16/10 Superdex 200 prep grade Column Chromatography

A sample was subjected to HiLoad 16/10 Superdex 200 prep grade (GE Healthcare) equilibrated with 200 mL of a buffer supplemented with 0.15 M NaCl. In the measurement of an absorbance at this time, a spectrophotometer U-300 manufactured by Hitachi Co., Ltd. was used, and the value of an absorbance when 5 minutes passed after the initiation of the reaction was measured.

### 2. Results and Discussion

The purification of the polyol oxidase was performed in the order of TOYOPEARL Butyl-650M column chromatography (hydrophobic column chromatography), TOYOPEARL DEAE-650M column chromatography (weak anion exchange column chromatography), Hiprep Q XL column chromatography (strong anion exchange column chromatography), and HiLoad 16/10 Superdex 200 prep grade column chromatography (gel filtration column chromatography).

The elution pattern of the TOYOPEARL Butyl-650M column chromatography is shown in Fig. 8. One activity peak was observed with 30% saturation ammonium sulfate, and therefore, the fractions of Fraction No. 7 to No. 12 having such an activity were combined and dialyzed, and the dialyzed solution was used for the subsequent column chromatography. Further, a small activity peak could also be observed in a fraction of around Fraction No. 45 obtained soon after 30% saturation ammonium sulfate was changed to 20% saturation ammonium sulfate. This is considered that the enzyme which could not be eluted with 30% saturation ammonium sulfate was eluted.

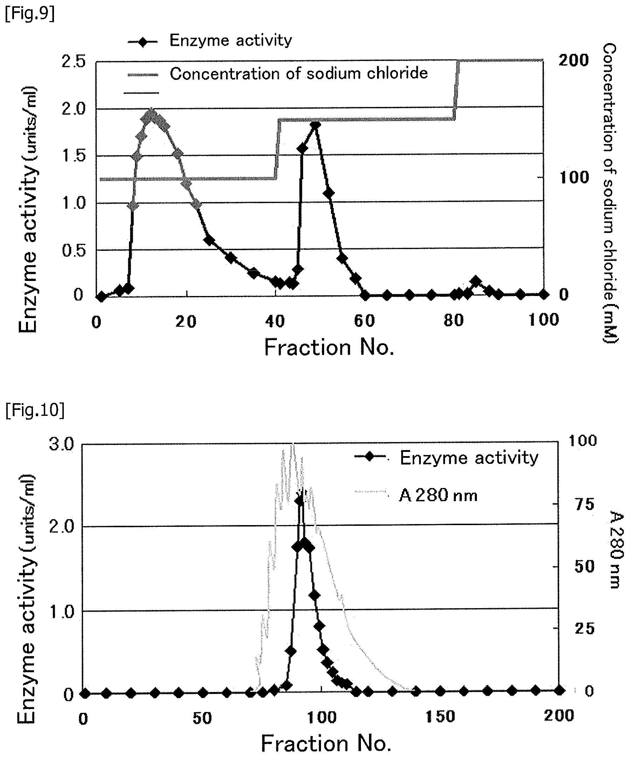

The elution pattern of the TOYOPEARL DEAE-650M column chromatography is shown in Fig. 9. Two activity peaks were observed in the fractions of Fraction No. 8 to No. 22 eluted with 100 mM sodium chloride and in the fractions of Fraction No. 40 to No. 52 eluted with 150 mM sodium chloride. However, when the specific activities were compared, the activity in the fractions of Fraction No. 8 to No. 22 was higher than the other, and therefore, these fractions were combined and dialyzed. Thereafter, the dialyzed solution was concentrated to about 1 mL by using Amicon Ultra-15 (manufactured by Millipore Co., Ltd.), and the resulting concentrate was used for Hiprep Q XL column chromatography. Since two peaks were observed, it is considered that an isozyme or a different enzyme may exist.

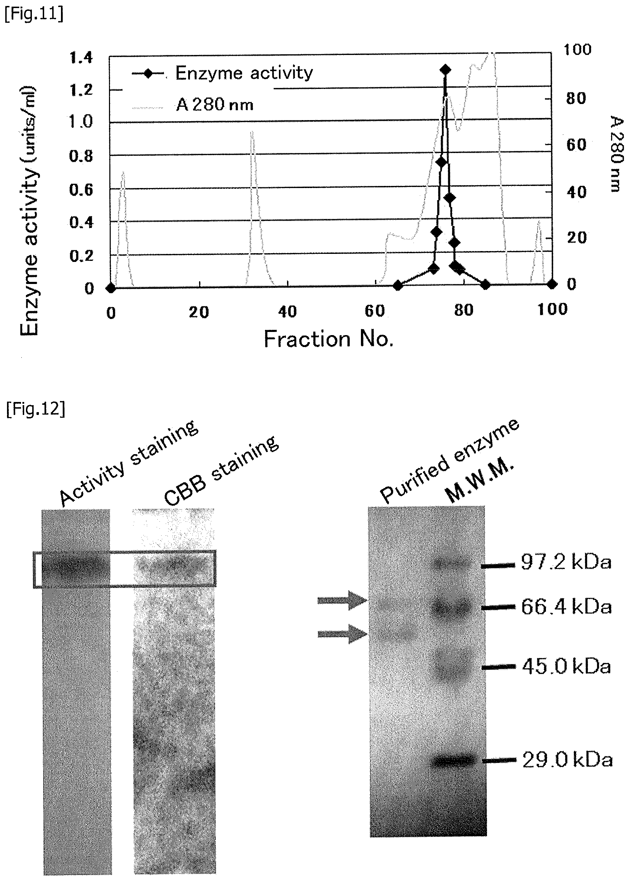

By the subsequently used Hiprep Q XL column chromatography, an elution pattern as shown in Fig. 10 was obtained, and one activity peak was observed. The fractions of Fraction No. 89 to No. 97, in which a high activity was observed, were combined and dialyzed. Thereafter, the dialyzed solution was concentrated to about 1 mL by using Amicon Ultra-15 (manufactured by Millipore Co., Ltd.), and the resulting concentrate was subjected to HiLoad 16/10 Superdex 200 prep grade column chromatography. By the HiLoad 16/10 Superdex 200 prep grade column chromatography, an elution pattern as shown in Fig. 11 was obtained. Further, the quantitative determination of a protein by measuring an absorbance at 280 nm was performed after the sample was subjected to Hiprep Q XL column chromatography in the purification process. This is because a brown dye was contained in the crude enzyme solution, and the sample was colored until the sample was subjected to TOYOPEARL DEAE-650M column chromatography. A purification table is shown in Table 12. As a result of the purification, the yield was about 0.1%, and the purification factor was about 3.0 times.

**[Table 12]**

| | Total protein amount (mg) | Specific activity (units/mg) | Total activity (units) | Yield (%) | Purification factor |
|---|---|---|---|---|---|
| Crude enzyme solution | 1800 | 22 | 40000 | 100 | 1.0 |
| Ammonium sulfate fraction (50 to 70%) | 160 | 46 | 7500 | 18 | 2.1 |
| TOYOPEARL Butyl-650M | 15 | 170 | 2500 | 6.0 | 7.6 |
| TOYOPEARL DEAE-650M | 63 | 180 | 1200 | 2.9 | 8.4 |
| Hiprep Q XL | 0.94 | 160 | 150 | 0.37 | 7.2 |
| HiLoad 16/10 Superdex 200 prep grade | 0.70 | 65 | 45 | 0.11 | 3.0 |

### [7. Analysis of Properties of Polyol Oxidase Derived from Strain Belonging to Genus Penicillium (A Strain)]

The properties of the polyol oxidase were analyzed using the purified enzyme solution obtained by the above-described methods.

### 1. Experimental Method

### 1.1 Purity Assay

A purity assay was performed by performing Native-PAGE followed by activity staining and GBB staining.

### 1.2 Molecular Weight and Examination of Subunit Structure

As described above, since HiLoad 16/10 Superdex 200 prep grade column chromatography (gel filtration column chromatography) was used in the final stage of the purification, also the calculation of the molecular weight was carried out simultaneously. The molecular weight of the enzyme was measured by using cytochrome C, carbonic anhydrase, albumin, and alcohol dehydrogenase from the Gel Filtration Molecular Weight Markers manufactured by SIGMA Co. , Ltd. as the molecular weight markers. Further, according to the results of SDS-PAGE of the purified enzyme, the subunit structure was assayed.

### 1.3 Examination of Temperature Stability

As the composition of the reaction solution, substantially the same composition as shown in Table 11 was employed. The examination was performed at 10°C, 20°C, 30°C, 40°C, 50°C, and 60°C. As the substrate, D-mannitol was used, and an increase in absorbance at 420 nm caused by the reaction under the conditions of 30°C was measured using a spectrophotometer U-2810 manufactured by Hitachi Co., Ltd. The reaction solution was prepared so that the total amount thereof was 1 mL, and the amount of the enzyme required to increase the absorbance at 420 nm by 1.0 for 1 minute was defined as "1 unit".

### 1.4 Examination of pH Stability

Each of buffers at various pH values was mixed with the enzyme to give a final concentration of 0. 1 M, and the resulting mixture was left to stand on ice for 15 hours, and then, the residual enzyme activity was measured. As the buffers, citrate buffers at pH 2.0, 3.0, 4.0, 5.0, and 6.0, potassium phosphate buffers at pH 6. 0, 7.0, and 8. 0, glycine-NaOH buffers at pH 8.0 and 9.0, and Tris-HCl buffers at pH 8.0, 9.0, and 10.0 were used. The measurement of the activity of this enzyme was performed by using D-mannitol as a substrate and measuring an increase in absorbance at 420 nm caused by the reaction under the conditions of 30°C using a spectrophotometer U-2010 manufactured by Hitachi Co., Ltd. Further, in the enzyme in the reaction solution, each of the buffers at various pH values added for testing the stability was contained. In order to perform the reaction at a uniform pH in all the cases, to the reaction solution, the potassium phosphate buffer (pH 8.0) was added in a larger amount than in the case of the common composition of the reaction solution so as to stabilize the reaction under the conditions of pH 8.0 (Table 13).

**[Table 13]**

| | |
|---|---|
| 1 M potassium phosphate buffer (pH 8.0) | 100 µL |
| Peroxidase (10 units/mL) | 100 µL |
| 10 mM ABTS | 100 µL |
| 0.5 M D-mannitol | 100 µL |
| Enzyme solution | 100 µL |
| H₂O | 500 µL |
| Total 1 mL | |

### 1.5 Examination of Optimum Reaction Temperature

As the composition of the reaction solution, the same composition as in the examination of the temperature stability was employed. An increase in absorbance at 420 nm in 10 minutes at 10°C, 20°C, 30°C, 40°C, 50°C, and 60°C was measured, respectively. In the measurement of the absorbance, a spectrophotometer U-3208 manufactured by Hitachi Co., Ltd. was used.

### 1.6 Examination of Optimum Reaction pH

The composition of the reaction solution is shown in Table 14. As the buffers at various pH values, the same buffers as used in the examination of the pH stability were used. The reaction solution was prepared so that the total amount thereof was 1 mL, and the concentration of the buffer in the reaction solution was adjusted to give a final concentration of 50 mM. In the measurement of the activity, an increase in absorbance at 420 nm caused by the reaction under the conditions of 30°C was measured using a spectrophotometer U-2010 manufactured by Hitachi Co., Ltd. Further, in a reaction solution, a peroxidase which is an enzyme was contained. Therefore, the peroxidase was added in a larger amount than usual so as to avoid the effect of this peroxidase on the pH.

**[Table 14]**

| | |
|---|---|
| Peroxidase (100 units/mL) | 100 µL |
| 10 mM ABTS | 100 µL |
| 0.5 M D-mannitol | 100 µL |
| Enzyme solution | 100 µL |
| in 1 mL of each 50 mM buffer | |

### 2. Results and Discussion

When the properties of the present enzyme were examined, the following results were obtained.

### 2.1 Purity Assay

As shown in Fig. 12, an almost single protein band was detected by Native-PAGE, and the position of the band coincided with the position of the band which could be confirmed by the activity staining. These results suggested that the present enzyme was substantially uniformly purified. However, the reason why the purification factor was as extremely low as 3 times although the enzyme was substantially uniformly purified is considered that the enzyme was inactivated during the purification process due to the effect of a protease and so on. Therefore, it is considered that some improvement such as use of a protease inhibitor is needed for the purification of the present enzyme.

### 2.2 Molecular Weight and Examination of Subunit Structure

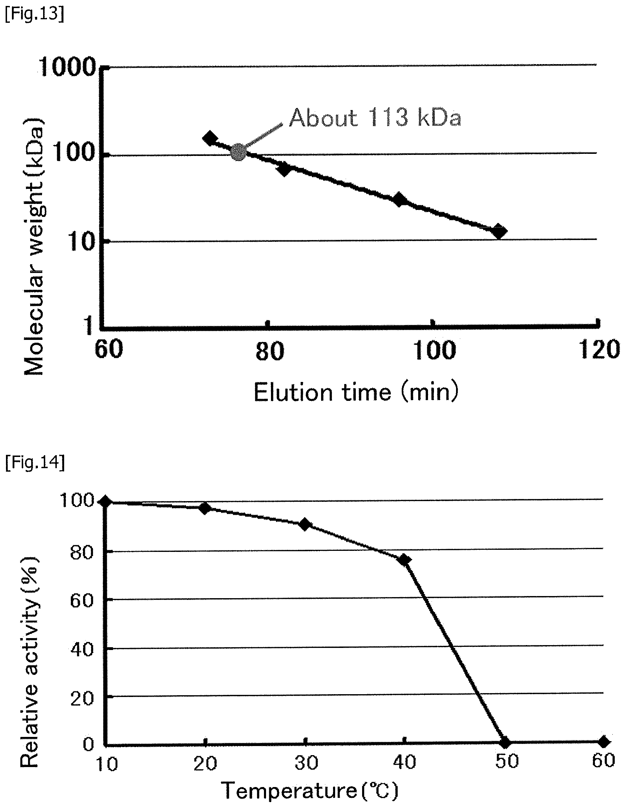

As shown in Fig. 13, the molecular weight of the present enzyme was calculated to be about 113 kDa by the gel filtration column chromatography, and two bands were detected at positions of about 50 kDa and 60 kDa by SDS-PAGE. These results suggested that the present enzyme is a heterodimeric enzyme composed of subunits having molecular weights of about 50 kDa and 60 kDa, respectively. There have been no reports of a heterodimeric enzyme among the known polyol oxidases, and therefore, it can be said that the novelty of this enzyme is high.

### 2.3 Examination of Temperature Stability

When the stability of the present enzyme was examined at temperatures between 10°C and 60°C, it was revealed that as shown in Fig. 14, the present enzyme is stable at 30°C or lower. Further, the enzyme has a residual activity of about 80% at 40°C, but the activity was completely lost at 50°C or higher.

### 2.4 Examination of pH Stability

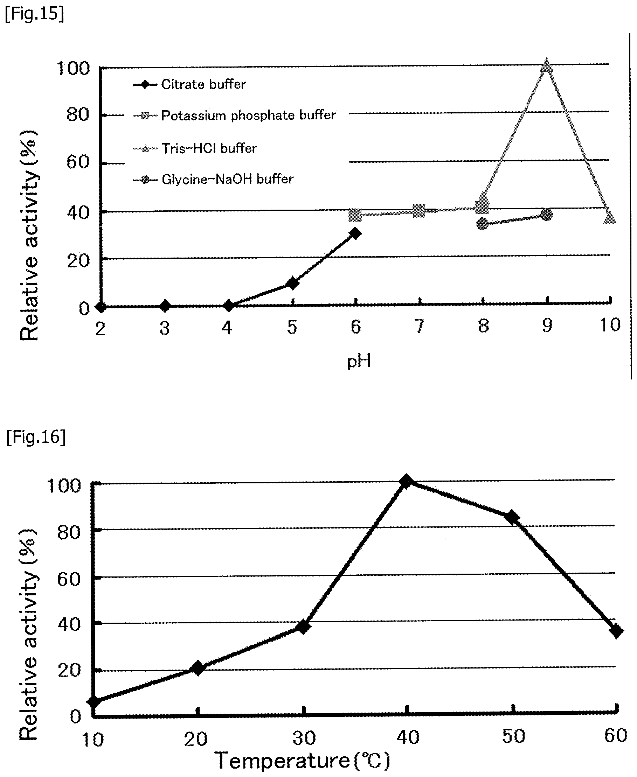

When the enzyme activity was measured at pH values between 2.0 and 10.0, it was found that as shown in Fig. 15, the enzyme was stable at a pH of 6.0 or higher. Further, the enzyme activity in a Tris-HCl buffer at pH 9. 0 was significantly high, and was about two times higher than the enzyme activity in a glycine-NaOH buffer at the same pH of 9.0. This suggested that the Tris-HCl buffer has some influence on the activity of the present enzyme.

### 2.5 Examination of Optimum Reaction Temperature

When the enzyme activity was measured at temperatures between 10°C and 60°C as shown in Fig. 16, it was found that the optimum reaction temperature is 40°C.

### 2.6 Examination of Optimum Reaction pH

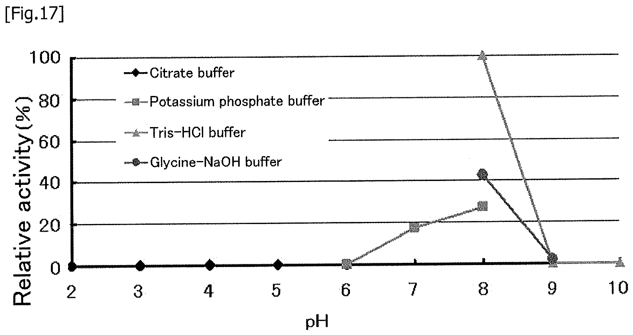

When the measurement for the reaction in each of the enzyme reaction solutions at pH values between 2.0 and 10.0 was performed as shown in Fig. 17, it was found that pH 8.0 is the optimum reaction pH. Further, no activity was detected at a pH of 6.0 or lower. Further, in the same manner as the examination of pH stability, the enzyme activity in a Tris-HCl buffer at pH 8. 0 was significantly high, and was about two times higher than the enzyme activity in a glycine-NaOH buffer at the same pH of 8.0. This suggested that the Tris-HCl buffer has some influence on the activity of the present enzyme. Further, an abrupt decrease in the activity was observed at pH 9.0. It is considered that this is because the peroxidase was added in a larger amount than the common composition of the reaction solution so as to prevent the effect of the peroxidase on the pH, however, the activity of the peroxidase abruptly decreases at a pH of 9.0 or higher, and therefore, the activity of the present enzyme was affected by the peroxidase after all.

### 2.7 Comparison of Properties between the Present Oxidase and Known Oxidases

A comparison of the enzymatic and chemical protein properties was made between the present enzyme and known polyol oxidases and oxidases produced specifically in a wheat bran culture medium.

The properties of known polyol oxidases and the properties of the present enzyme are shown in Table 15. The present enzyme has a very large molecular weight and lower temperature stability than the other polyol oxidases. Further, there have been no reported cases that polyol oxidases are heterodimeric enzymes. These points more strongly supported the novelty of the present enzyme. Further, the properties of known oxidases produced specifically in a wheat bran culture medium and the properties of the present enzyme are shown in Table 16. The present enzyme and the polyol oxidases produced specifically in a wheat bran culture medium have similar pH stability and optimum reaction temperature, however, in the same manner as the case where the comparison was made with the known polyol oxidases, the present enzyme has lower temperature stability than the other oxidases. However, a glutathione oxidase and a glycerol oxidase derived from a microorganism belonging to the genus Penicillium in the same manner as the present enzyme are heterodimeric enzymes, and therefore are similar in the respect that they have a large molecular weight. Due to this, it is considered that microorganisms belonging to the genus Penicillium extracellularly produce and secrete heterodimeric enzymes more than microorganisms belonging to the other genera in a wheat bran culture medium. Further, in the case of the glycerol oxidase derived from a microorganism belonging to the genus Penicillium, since this enzyme is an enzyme binding to a cell surface, there has been reported that by adding a surfactant to a buffer when extracting the enzyme, the enzyme activity was extremely increased.

**[Table 15]**

| | Molecular weight | Structure | Temperature stability | pH stability | Optimum reaction temperature | Optimum reaction pH |
|---|---|---|---|---|---|---|
| Polyol oxidase derived from Penicillium sp. | About 113 kDa | Heterodimer | 30°C or lower | pH 6.0 or higher | 40°C | pH 8.0 |
| Xylitol oxidase derived from Streptomyces sp. IKD472¹⁵⁾ | 43 kDa | Monomer | 65°C or lower | pH 5.5 to 10.5 | 55°C | pH 7.5 |
| Sorbitol oxidase derived from Streptomyces sp. H-7775¹²⁾ | 45 kDa | Monomer | 55°C or lower | N.D. | N.D. | pH 6.5 to 7.5 |
| Alditol oxidase derived from Streptomyces coelicolor¹⁹⁾ | 45.1 kDa | Monomer | N.D. | N.D. | N.D. | N.D. |
| Mannitol oxidase derived from Helix aspersa (snail)²⁰⁾ | N.D. | N.D. | N.D. | N.D. | N.D. | pH 8.0 to 8.5 |
| Mannitol oxidase derived from Arion ater (slug)²¹⁾ | About 68 kDa | N.D. | N.D. | N.D. | N.D. | N.D. |
| Sorbitol oxidase derived from apple leaves²²⁾ | N.D. | N.D. | N.D. | N.D. | N.D. | pH 4.0 |

| | | | | | | |
|---|---|---|---|---|---|---|
| N.D.: not determined | | | | | | |

**[Table 16]**

| | Molecular weight | Structure | Temperature stability | pH stability | Optimum reaction temperature | Optimum reaction pH |
|---|---|---|---|---|---|---|
| Polyol oxidase derived from Penicillium sp. | About 113 kDa | Heterodimer | 30°C or lower | pH 6.0 or higher | 40°C | pH 8.0 |
| Glutathione oxidase derived from Penicillium sp.⁵⁾ | 95 kDa | Heterodimer | 45°C or lower | pH 5.2 to 8.6 | N.D. | pH 7.0 to 7.8 |
| Glycerol oxidase derived from Penicillium sp.¹⁰⁾ | 400 kDa | Heterodimer | 40°C or lower | pH 5.5 to 6.5 | 45°C | pH 6.0 to 7.0 |
| Glucooligosaccharide oxidase derived from Acremonium strictum ⁹⁾ | 61 kDa | Monomer | 50°C or lower | pH 5.0 to 11.0 | 50°C | pH 10.0 |
| Cellooligosaccharide oxidase derived from Sarocladium oryzae²³⁾ | 55 kDa | Monomer | 40°C or lower | pH 5.0 to 10.0 | 45°C | pH 10.0 |

| | | | | | | |
|---|---|---|---|---|---|---|
| N.D.: not determined | | | | | | |

### [8. Substrate Specificity of Enzyme]

### 1. Substrate Specificity

The substrate specificity of the present enzyme determined by using a partially purified enzyme was as shown in Table 17 below. Among the polyols used in the assay, the activity of the enzyme was high for D-arabitol, erythritol, D-mannitol, and D-sorbitol, which are listed in descending order of activity, and a high activity was not observed for the other polyols.

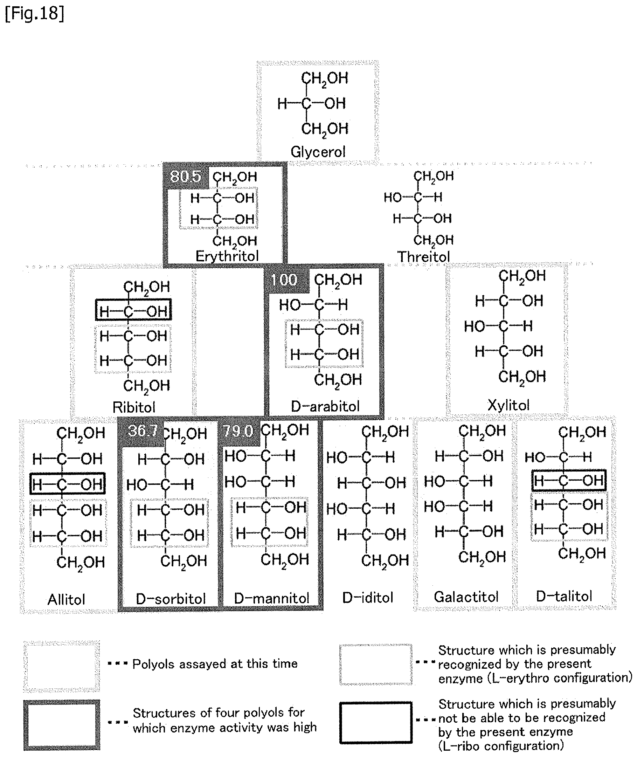

The structures of the D-forms of polyols are shown in Fig. 18. When the structures of all the polyols used in the assay were compared, a common structure among the four polyols for which a high activity was observed was confirmed. As shown in Fig. 18, when the polyols were represented by the Fischer's structural formula, it was suggested that the present enzyme specifically recognizes the L-erythro configuration in which the OH groups at positions 2 and 3 are located on the right-hand side. However, this configuration is also seen in ribitol, allitol, and D-talitol, for which the enzyme activity was low. When comparing the structures of these polyols with the structures of the four polyols for which the enzyme activity was high, it is found that a difference therebetween is the structure of the L-ribo configuration in which the OH group at position 4 is located on the right-hand side. Due to this, it was considered that the present enzyme cannot recognize the structure in which the OH group at position 4 is in the L-ribo configuration, and thus, an oxidation reaction does not occur for these ribitol, allitol, and D-talitol.

Both of the substrate specificity and the substrate recognition mechanism of the present enzyme are different from those of conventionally reported polyol oxidases, and therefore, the present enzyme is a novel oxidase.

**[Table 17]**

| Substrate | Relative activity (%) |
|---|---|
| D-arabitol | 100 |
| Erythritol | 80.5 |
| D-mannitol | 79.0 |
| D-sorbitol | 36.7 |
| Xylitol | 1.2 |
| D-talitol | 0.3 |
| Allitol | 0.45 |
| L-talitol | 0.3 |
| Glycerol | 0.18 |
| Ribitol | - |
| L-arabitol | - |
| L-sorbitol | - |
| L-mannitol | - |
| Galactitol | - |

### 2. Assay for Reaction Product by HPLC

The reaction products of the polyol oxidase were analyzed by HPLC. As a result, products when using D-mannitol, D-arabitol, and D-sorbitol as substrates were identified. Erythritol is a tetrose, and it was difficult to identify the product at this stage, and therefore, the analysis was not performed at this time. It was confirmed that from D-mannitol, mannose was produced. Although an optical activity was not measured, when considering that D-mannitol was used as the substrate, the product is structurally considered to be D-mannose.

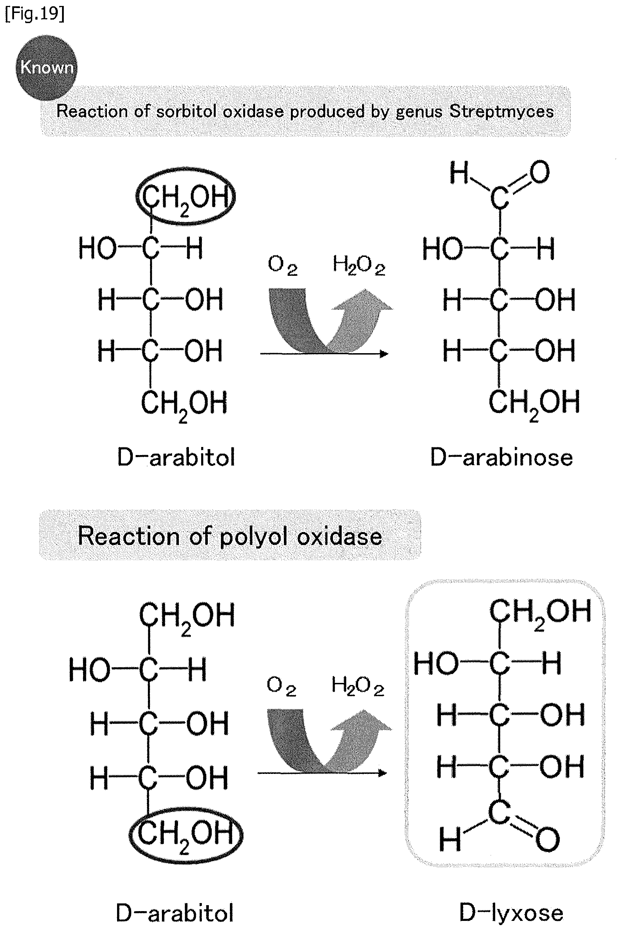

According to the results of the HPLC analysis of the enzyme reaction solution when using D-arabitol as a substrate, it was revealed that the product from D-arabitol is lyxose. Although an optical activity of the product was not measured also in this case, the reaction product is structurally considered to be D-lyxose. The structural formula of this reaction is shown in Fig. 19. When using a polyol oxidase which has been reported so far, D-arabinose is produced by oxidizing the hydroxy group at position 1 of D-arabitol. Here, a sorbitol oxidase produced by a microorganism belonging to the genus Streptmyces is taken as an example. On the other hand, it was suggested that the present enzyme catalyzes the oxidation of the hydroxy group at position 6 of D-arabitol, whereby D-lyxose which is a rare sugar is produced. Here, according to the results of HPLC, one peak was observed immediately upstream of the peak of D-arabitol, however, this peak was confirmed also before performing the enzymatic reaction and in the case of using any substrate. Therefore, this peak was considered to be a component other than sugars derived from the microbial cells.

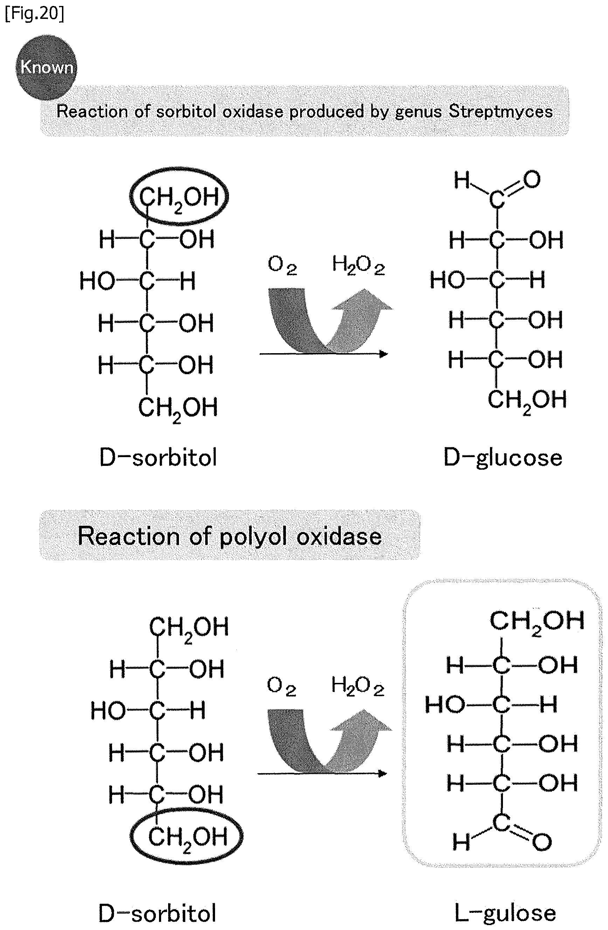

Next, according to the results of the HPLC analysis of the reaction product when using D-sorbitol as a substrate, it was confirmed that gulose was produced from D-sorbitol. Although an optical activity was not measured also in this case, since gulose was produced from D-sorbitol, structurally, L-gulose is considered to be produced. The structural formula of this reaction is shown in Fig. 20. Also in Fig. 20, a sorbitol oxidase which is a known polyol oxidase is taken as an example. By combining the previously shown results obtained when using D-mannitol and D-arabitol as the substrates with these results, the present enzyme is considered to be an enzyme which oxidizes the hydroxy group at position 6 of a polyol. There have been no reports of an enzyme which oxidizes the hydroxy group at position 6 of a polyol among the polyol oxidases having been reported so far, and therefore, it was suggested again that the present enzyme is a novel oxidase.

Here, according to the fact that the present enzyme catalyzes the oxidation of the hydroxy group at position 6 of a polyol, an oxidation reaction using as a substrate, erythritol which was not analyzed by HPLC at this time was inferred. When the hydroxy group at position 6 of erythritol is oxidized, L-erythrose is expected to be produced. This strongly suggested that by the oxidation reaction of the present enzyme, the following three rare sugars: D-lyxose, L-gulose, and L-erythrose are produced.

When the conventional production methods for these rare sugars were examined, it was found that D-lyxose is produced from D-glucose through D-arabitol and D-xylose by a multistep reaction (NPL 2). Moreover, since an isomerase is used, it is difficult to say that D-lyxose to be produced is obtained in high yield. Also L-gulose is produced from L-sorbose by using an isomerase. As for L-erythrose, erythritol is subjected to oxidative fermentation using a membrane-bound meso-erythritol dehydrogenase present on the outer surface layer of the cell membrane of a strain belonging to the genus Gliconobacter, thereby producing L-erythrulose, and thereafter L-erythrose is produced by using an isomerase. As described above, the production of such rare sugars includes a multistep reaction system, and also the reaction uses an isomerase, and therefore, a yield of 100% cannot be expected. In this respect, the present enzyme catalyzes a one-step reaction and also the oxidation reaction is an irreversible reaction, and therefore, it is expected that the present enzyme enables the mass production of such rare sugars in a yield of almost 100%.

### [9. Identification of Polyol Oxidase-Producing Microorganism]

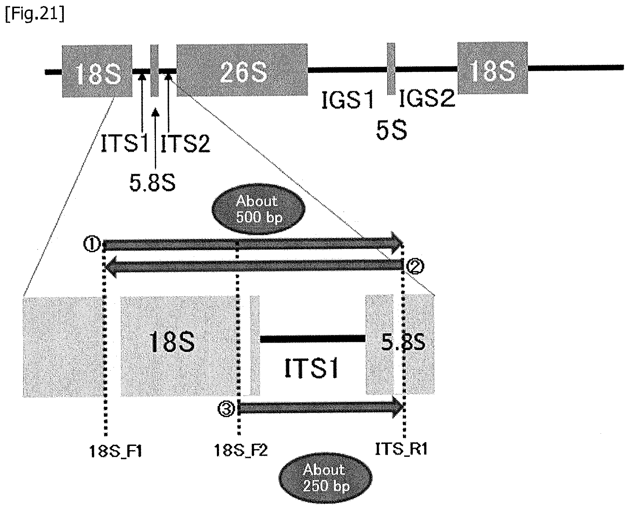

In order to identify a polyol oxidase-producing microorganism, the sequence of 18S rDNA was intended to be determined. The 18S rDNA is a structure specifically seen in fungal microorganisms and is not present in Actinomyces or bacteria. The structure of the 18S rDNA is shown in Fig. 21.

### 1. Experimental Method

### 1.1 Preparation of Primers

The nucleotide sequences of the primers prepared in this study are shown in Table 18.
(1) The preparation of 18S_F1 primer was performed with reference to the evaluation of gene indices for identifying Byssochlamys spp. (NPL 3 and NPL 4).
(2) The preparation of 18S_F2 and ITS_R1 primers was performed with reference to the homepage of BEX Co., Ltd. (http://www.bexnet.co.jp/product/microbialprimer.html).

**[Table 18]**

| | |
|---|---|
| ① 18S_F1: | GGGGTAAGAGCATTGCAATTATTGC |
| ② 18S_F2: | GTAACAAGGTYTCCGT |
| ③ ITS_R1: | CGTTCTTCATCGATG |

18S_F1 was prepared so that it has a sequence homologous to a region of a sequence which is located downstream of the 18S rDNA and common to a wide range of fungi; 18S_F2 was prepared so that it has a sequence homologous to a region of a sequence of the 18S rDNA located immediately upstream of the ITS region; and ITS_R1 was prepared so that it has a sequence homologous to a region located in the middle of 5.8S (Fig. 21).

### 1.2 PCR and Sequence Reaction

### (1) Preparation Method for total DNA

1) Liquid-cultured microbial cells were placed in a mortar, and liquid nitrogen was added thereto, and then, the microbial cells were ground and transferred to a microtube.
2) 600 µL of a 2% CTAB solution was added thereto and mixed therewith by inverting the microtube repeatedly.
3) The tube was transferred to a heat block, which was heated to 65°C, and incubated for 30 minutes, followed by centrifugation at 12,000 rpm for 10 minutes.
4) The supernatant was collected, and an equal amount of a mixture of chloroform/isoamyl alcohol (24:1) was added thereto, and the resulting mixture was gently stirred for 5 minutes.
5) The resulting mixture was centrifuged at 12,000 rpm for 15 minutes, and then, an upper aqueous phase was collected.
6) The procedures described in 4) and 5) were repeated once, and an aqueous phase was transferred to a new tube.
7) 1 to 1. 5 volume of a 1% CTAB solution was added thereto and mixed therewith by inverting the tube repeatedly. Thereafter, the resulting mixture was left to stand at room temperature for 1 hour, followed by centrifugation at 8,000 rpm for 10 minutes.
8) The supernatant was discarded, and 400 µL of 1 M CsCl was added to the tube, and the residue was completely dissolved therein.
9) 800 µL of 100% ethanol was added thereto and mixed therewith by inverting the tube repeatedly. Thereafter, the resulting mixture was left to stand at -20°C for 20 minutes or more, followed by centrifugation at 12, 000 rpm for 5 minutes.
10) The supernatant was discarded, and 400 µL of 70% ethanol was added to the precipitate, followed by centrifugation at 12,000 rpm for 5 minutes.
11) The supernatant was discarded, and the precipitate was air-dried using a vacuum dryer. Then, the dried precipitate was dissolved in 20 µL of a TE buffer and treated at 37°C for 1 hour.
12) 100 µL each of phenol and chloroform was added thereto, followed by centrifugation at 12,000 rpm for 10 minutes, and the supernatant was collected.
13) An equal amount of chloroform was added thereto, followed by centrifugation at 12,000 rpm for 10 minutes, and the supernatant was collected.
14) Ethanol precipitation was performed, followed by centrifugation at 12,000 rpm for 10 minutes, and thereafter, the resulting precipitate was dissolved in 30 µL of a TE buffer.

### (2) PCR

PCR was performed using the 18S_F1 primer and the ITS_R1 primer, and also using the total DNA of the polyol oxidase-producing microorganism obtained in (1) as the template. The conditions for the PCR were as follows: denaturation was performed at 95°C for 3 minutes, followed by 33 cycles, each cycle consisting of heat treatments at 95°C, 50°C, and 75°C. The PCR reaction solution shown in Table 19 was used.

**[Table 19]**

| | |
|---|---|
| Template DNA | 0.8 µL |
| 10 x Ex Taq™ buffer | 1.0 µL |
| dNTP Mixture (2.5 mM) | 0.4 µL |
| 18S_F1 (10 pmol/µL) | 0.8 µL |
| ITS_R1 (10 pmol/µL) | 0.8 µL |
| Ex Taq polymerase | * |
| H₂O | 6.2 µL |

| | |
|---|---|
| * Ex Taq was attached to a tip end and suspended in a microtube. | |

### (3) Sequence Reaction

Fragments amplified by the PCR were electrophoresed, followed by gel extraction. Thereafter, a sequence reaction was performed using the respective primers (Fig. 21). The conditions for the sequence reaction were as follows: 25 cycles, each cycle consisting of heat treatments at 95°C, 50°C, and 75°C. The composition of the sequence reaction solution is shown in Table 20.

**[Table 20]**

| | |
|---|---|
| Template DNA | 2.0 µL |
| Cycle sequence Mix | 2.5 µL |
| Primer (2 pmol/µL) | 5.5 µL |

### 2. Results and Discussion

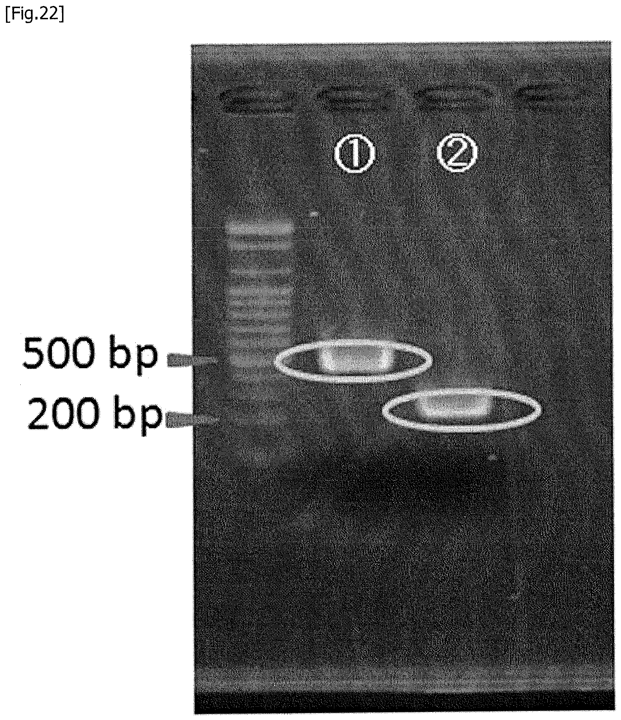

As a result of performing electrophoresis in an agarose gel after the PCR, it was confirmed that a fragment having a target size was amplified (Fig 22). Accordingly, it was revealed that this microorganism is a eukaryotic filamentous fungus.

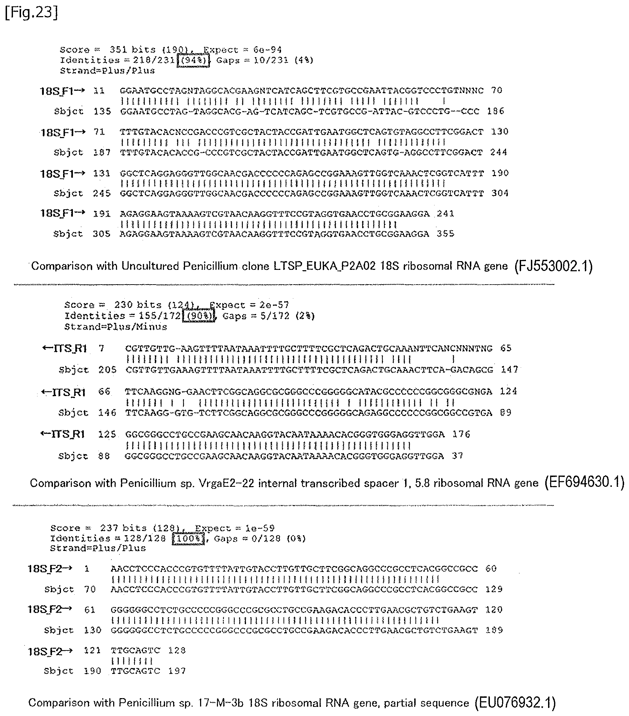

After the sequence reaction, an analysis was performed. As a result, a 250-bp base sequence was found by the sequence reaction using the 18S_F1, and a 180-bp base sequence was found by the reaction using the ITS_R1. Further, (2) a 159-bp sequence was determined by the sequence reaction using the 18S_F2. As a result of the respective analyses, the full length of the PCR-amplified fragment of the polyol oxidase-producing microorganism could not be decoded. However, when a microorganism having a high homology to the sequences obtained in the respective cases was searched, the 250-bp base sequence obtained by using the 18S_F1 primer had a homology of 94% to the genus Penicillium (Fig. 23) and a homology of 93% to the genus Aspergillus. The 180-bp base sequence obtained by using the ITS_R1 primer had a homology of 90% to a Penicillium microorganism (Fig. 23). Further, the 159-bp base sequence obtained by using the 18S_F2 had a homology of 100% to the genus Penicillium and the base sequences were identical to each other (Fig. 23). Accordingly, it is strongly suggested that this microorganism is a microorganism belonging to the genus Penicillium.

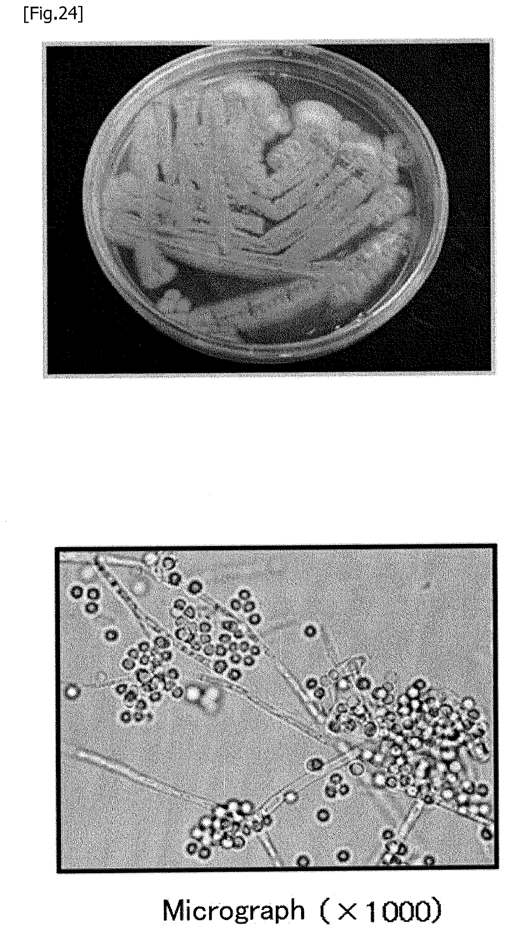

In Fig. 24, a photograph of the polyol oxidase-producing microorganism and a photograph taken by a light microscope (x 1000) are shown. Also based on these morphologies, it was suggested that this microorganism is a microorganism belonging to the genus Penicillium.

Among the polyol oxidases produced by microorganisms which have been reported so far, there have been almost no reports of an extracellular enzyme. Also from this point, the present enzyme is considered to be a highly novel enzyme.

### Industrial Applicability

The present invention provides a novel polyol oxidase, and particularly, the polyol oxidase is characterized by an ability to produce rare sugars from substrates. Rare sugars are sugars which do not exist at all or exist in a very small amount in nature. Although there were many unclear points with respect to the production techniques, physiological actions, chemical properties, etc. of rare sugars, recently, the mass production techniques for some rare sugars have been established, and also the physiological activities thereof have been elucidated, and thus, the practical application thereof in a wide field of sweeteners, agricultural chemicals, reagents, industrial materials, etc. has been expected. The provision of the polyol oxidase of the present invention responds to such expectations in the industry, and will be a means useful for new development of the measurement of rare sugars and the efficient production method therefor.

## Claims

1. A polyol oxidase, which is derived from Penicillium sp. KU-1, deposited under Accession Number: NITE BP-1156, a microorganism belonging to the genus Penicillium, and is specified by the properties listed in the following (a) to (e):
(a) it specifically recognizes the structure of a polyol in which the OH groups at positions 2 and 3 are in the L-erythro configuration and reacts therewith, and it cannot recognize the structure of a polyol in which the OH group at position 4 is in the L-ribo configuration and does not react therewith;
(b) it has an activity to oxidize a sugar alcohol selected from D-arabitol, erythritol, D-mannitol, and D-sorbitol, which are listed in descending order of activity, to produce a corresponding aldose selected from D-lyxose, L-erythrose, D-mannose, and L-gulose,
(c) it is stable at a pH of 6.0 or higher and has an optimum reaction pH of from 7.0 to 9.0;
(d) it has an operating temperature of 50°C or lower and an optimum reaction temperature of 40°C; and
(e) it has a molecular weight of about 113 kDa.

2. The polyol oxidase according to claim 1, wherein the polyol oxidase is obtained by culturing the microorganism belonging to the genus Penicillium using wheat bran as a culture medium.

3. A method for producing a polyol oxidase, **characterized in that** a microorganism including Penicillium sp. KU-1, deposited under Accession Number: NITE BP-1156, having the ability to produce a polyol oxidase as defined in claim 1 or 2, is cultured in a wheat bran culture medium, thereby producing the polyol oxidase, and the polyol oxidase is collected from the obtained culture.

4. A method for producing an aldose selected from D-mannose, D-lyxose, L-gulose, and L-erythrose, **characterized in that** a polyol oxidase derived from Penicillium sp. KU-1, deposited under Accession Number: NITE BP-1156, as defined in claim 1 or 2, is allowed to act on a sugar alcohol selected from D-mannitol, D-arabitol, D-sorbitol, and erythritol, thereby producing a corresponding aldose selected from D-mannose, D-lyxose, L-gulose, and L-erythrose.

## Patentansprüche

1. Polyoloxidase, welche aus Penicillium sp. KU-1 stammt, hinterlegt unter der Eingangsnummer: NITE BP-1156, ein Mikroorganismus, der zum Genus Penicillium gehört, und die durch die in den folgenden (a) bis (e) aufgelisteten Eigenschaften spezifiziert ist:
(a) sie erkennt spezifisch die Struktur eines Polyols, in welchem die OH-Gruppen an den Positionen 2 und 3 in der L-Erythro-Konfiguration sind, und reagiert damit, und sie kann nicht die Struktur eines Polyols erkennen, in welchem die OH-Gruppe an der Position 4 in der L-Ribo-Konfiguration ist, und reagiert nicht damit;
(b) sie weist eine Aktivität auf, um einen Zuckeralkohol ausgewählt aus D-Arabitol, Erythritol, D-Mannitol und D-Sorbitol zu oxidieren, welche in der abnehmenden Reihenfolge der Aktivität aufgelistet sind, um die entsprechende Aldose ausgewählt aus D-Lyxose, L-Erythrose, D-Mannose und L-Gulose zu erzeugen,
(c) sie ist stabil bei einem pH von 6,0 oder höher und weist einen optimalen Reaktions-pH von 7,0 bis 9,0 auf;
(d) sie weist eine Arbeitstemperatur von 50°C oder niedriger und eine optimale Reaktionstemperatur von 40°C auf; und
(e) sie weist ein Molekulargewicht von etwa 113 kDa auf.

2. Polyoloxidase nach Anspruch 1, wobei die Polyoloxidase erhalten wird durch Kultivieren des zu dem Genus Penicillium gehörenden Mikroorganismus unter Verwendung von Weizenkleie als ein Kulturmedium.

3. Verfahren für die Herstellung einer Polyoloxidase, **dadurch gekennzeichnet, dass** ein Mikroorganismus einschließlich Penicillium sp. KU-1, hinterlegt unter der Eingangsnummer: NITE BP-1156, mit der Fähigkeit eine Polyoloxidase wie in Anspruch 1 oder 2 definiert zu erzeugen, in einem Weizenkleiekulturmedium kultiviert wird, dadurch die Polyoloxidase erzeugt wird und die Polyoloxidase aus der erhaltenen Kultur gewonnen wird.

4. Verfahren für die Herstellung einer Aldose ausgewählt aus D-Mannose, D-Lyxose, L-Gulose und L-Erythrose, **dadurch gekennzeichnet, dass** eine Polyoloxidase, die aus Penicillium sp. KU-1, hinterlegt unter Eingangsnummer: NITE BP-1156, stammt, wie in Anspruch 1 oder 2 definiert, auf einen Zuckeralkohol ausgewählt aus D-Mannitol, D-Arabitol, D-Sorbitol und Erythritol wirkt, dadurch wird eine entsprechende Aldose ausgewählt aus D-Mannose, D-Lyxose, L-Gulose und L-Erythrose hergestellt.

## Revendications

1. Polyol oxydase qui dérive de Pénicillium sp. KU-1 déposé avec le numéro d'accès NITE BP-1156, un microorganisme appartenant au genre Pénicillium, et est spécifiée par les propriétés listées dans les paragraphes (a) à (e) suivants :
(a) elle reconnaît spécifiquement la structure d'un polyol dans lequel les groupes OH aux positions 2 et 3 sont en configuration L-érythro, et réagit avec celui-ci, et ne peut pas reconnaître la structure d'un polyol dans lequel le groupe OH en position 4 est en configuration L-ribo, et ne réagit pas avec celui-ci ;
(b) elle a une activité d'oxydation d'un alcool de sucre choisi parmi le D-arabitol, l'érythritol, le D-mannitol et le D-sorbitol, qui sont énumérés dans l'ordre descendant d'activité, pour produire un aldose correspondant choisi parmi le D-lyxose, le L-érythrose, le D-mannose, et le L-gulose,
(c) elle est stable à un pH de 6,0 ou plus et a un pH réactionnel optimal de 7,0 à 9,0 ;
(d) elle a une température opérationnelle de 50 °C ou moins et une température réactionnelle optimale de 40 °C ; et
(e) elle a un poids moléculaire d'environ 113 kDa.

2. Polyol oxydase selon la revendication 1, laquelle polyol oxydase est obtenue par culture du microorganisme appartenant au genre Pénicillium utilisant du son de blé en tant que milieu de culture.

3. Procédé pour produire une polyol oxydase, **caractérisé en ce qu'**un microorganisme comprenant Pénicillium sp. KU-1 déposé avec le numéro d'accès NITE BP-1156, capable de produire une polyol oxydase telle que définie dans la revendication 1 ou 2, est cultivé dans un milieu de culture à base de son de blé, en produisant ainsi la polyol oxydase, et la polyol oxydase est collectée à partir de la culture obtenue.

4. Procédé pour produire un aldose choisi parmi le D-mannose, le D-lyxose, le L-gulose et le L-érythrose, **caractérisé en ce qu'**une polyol oxydase dérivée de Penicillium sp. KU-1 déposé avec le numéro d'accès NITE BP-1156, telle que définie dans la revendication 1 ou 2, est laissée à agir sur un alcool de sucre choisi parmi le D-mannitol, le D-arabitol, le
D-sorbitol, et l'érythritol, en produisant ainsi un aldose correspondant choisi parmi le D-mannose, le D-lyxose, le L-gulose et le L-érythrose.
